# EUROPEAN PATENT SPECIFICATION

(11) **EP 1 670 940 B1**
(45) Date of publication and mention of the grant of the patent: **24.11.2010**
(21) Application number: 04765898.4
(22) Date of filing: 08.10.2004
(51) Int. Cl.: C12Q 1/68, G01N 33/68

(54) **METHOD FOR IDENTIFICATION OF SUITABLE FRAGMENTATION SITES IN A REPORTER PROTEIN**
VERFAHREN ZUR IDENTIFIZIERUNG GEEIGNETER FRAGMENTIERUNGSSTELLEN IN EINEM REPORTERPROTEIN
METHODE D IDENTIFICATION DE SITES DE FRAGMENTATION ADAPTES DANS UNE PROTEINE REPORTER

(30) Priority: 09.10.2003 US 510231 P
(43) Date of publication of application: 21.06.2006
(60) Divisional application: 10160678.8
(73) Proprietor: ECOLE POLYTECHNIQUE FEDERALE DE LAUSANNE, 1015 Lausanne (CH)
(72) Inventor: JOHNSSON, Kai, CH-1003 Lausanne (CH); TAFELMEYER, Petra, 75014 Paris (FR)
(74) Representative: Müller, Christoph Emanuel
(86) International application number: PCT/EP2004/011289
(87) International publication number: WO 2005/038050

(56) References cited:
- WO-A-99/49294
- US-A1- 2003 138 850
- DATABASE EMBL [Online] 21 December 1999 (1999-12-21), "pti1c.pk002.i20 chicken MDV infected T cell cDNA library Gallus gallus cDNA clone pti1c.pk002.i20 5' similar to n-(5'-phosphoribosyl)-anthranilate isomerase,, mRNA sequence." XP002313063 retrieved from EBI accession no. EM_PRO:AW239796 Database accession no. AW239796
- DATABASE EMBL [Online] 6 June 1997 (1997-06-06), "H.sapiens telomeric DNA sequence, clone 9QTEL021, read 9QTELOO021.seq." XP002313064 retrieved from EBI accession no. EM_PRO:HS9QT021 Database accession no. HS9QT021
- DATABASE EMBL [Online] 4 September 2003 (2003-09-04), "Sequence 3 from patent US 5217864." XP002313065 retrieved from EBI accession no. EM_PRO:AR363478 Database accession no. AR363478
- JOHNSSON N ET AL: "SPLIT UBIQUITIN AS A SENSOR OF PROTEIN INTERACTIONS INV VIVO" PROCEEDINGS OF THE NATIONAL ACADEMY OF SCIENCES OF USA, NATIONAL ACADEMY OF SCIENCE. WASHINGTON, US, vol. 91, 1 October 1994 (1994-10-01), pages 10340-10344, XP002064564 ISSN: 0027-8424 cited in the application
- VAN CRIEKINGE WIM ET AL: "Yeast two-hybrid: State of the art" BIOLOGICAL PROCEDURES ONLINE, vol. 2, no. 1 Cited May 10, 2001, 4 October 1999 (1999-10-04), pages 1-38, XP002313062 ISSN: 1480-9222

## Description

The present invention is related to the field of methods for detecting the interaction of proteins via the use of fusion proteins, commonly referred to as split-protein sensors or two-hybrid assays.

The introduction of the yeast-two hybrid system by Fields and Song in 1989 was a milestone for the analysis of protein-protein interactions in living cells (cf. US 5,667,973 and Fields, S., and Song, O. (1989), Nature 340, 245-246). However, a major limitation of this classical two-hybrid system lies in its restriction to the detection of those protein-protein interactions that can be reproduced within the nucleus of a yeast cell. To overcome this restriction, an alternative to this two-hybrid method was introduced in 1994 by Johnsson and Varshavsky (cf. WO 95/29195 and Johnsson, N., and Varshavsky, A. (1994), Proc Natl Acad Sci U S A 91, 10340-10344). Here, the two interacting proteins are expressed as fusion proteins with an N- and a C-terminal fragment of ubiquitin. Upon interaction of the two proteins a quasi-native ubiquitin is formed and subsequently recognized by ubiquitin-specific proteases, resulting in the cleavage of a reporter protein from the C-terminal fragment of ubiquitin. The split-ubiquitin system allows for the detection of interactions between cytoplasmic as well as membrane proteins. Since the introduction of split-ubiquitin, a variety of other split-protein sensors has been developed, including pairs of fragments of dihydrofolate reductase (DHFR), β-galactosidase, β-lactamase, inteins, green fluorescent protein (GFP), cAMP cyclase, glycinamide ribonucleotide transformylase, aminoglycoside phosphotransferase, hygromycin B phosphotransferase, and luciferase (cf. Remy, I., and Michnick, S.W. (1999), Proc Natl Acad Sci U S A 96, 5394-5399; Rossi, F., Charlton, C.A., and Blau, H.M. (1997), Proc Natl Acad Sci U S A 94, 8405-8410; Galarneau, A., Primeau, M., Trudeau, L.E., and Michnick, S.W. (2002), Nat Biotechnol 20, 619-622; Wehrman, T., Kleaveland, B., Her, J.H., Balint, R.F., and Blau, H.M. (2002), Proc Natl Acad Sci U S A 99, 3469-3474; Ozawa, T., Nogami, S., Sato, M., Ohya, Y., and Umezawa, Y. (2000), Anal Chem 72, 5151-5157; Ozawa, T., Kaihara, A., Sato, M., Tachihara, K., and Umezawa, Y. (2001), Anal Chem 73, 2516-2521; Ghosh, I., Hamilton, A.D., and Regan, L. (2000), Journal of the American Chemical Society 122, 5658-5659). Among these systems only split-ubiquitin was successfully applied to screen for binding partners. Other sensors were used to monitor the interactions between selected pairs of proteins rather than to find new partners by a random library approach. Robust systems that can be used for identifying interaction partners at any location inside the cell and in different hosts are therefore still needed. Ideally the interaction-induced reassociation of such a split-protein sensor would provide the cell with a growth advantage thus allowing a selection for interacting proteins. However, generating new split-protein sensors is technically demanding as it depends critically on identifying suitable fragments that can reconstitute a native-like and active protein. The chosen fragmentation site has to fulfill at least the following criteria: (i) to yield two fragments that efficiently fold into quasi-native protein only when fused to two interacting proteins; (ii) not to significantly impair the activity of the reconstituted protein; (iii) to yield soluble protein fragments that are not readily degraded in vivo. In previous studies, the challenge of rationally finding such sites has been mostly tackled by trial and error.

It is thus an object of the present invention to overcome the above-mentioned drawbacks of the prior art, i.e. to provide a method for identification of suitable fragmentation sites in a reporter protein especially for use as a split-protein sensor, that is not limited by the above-mentioned drawbacks of rational design, and which especially allows for the identification of suitable fragmentation sites in a reporter protein even in the absence of any structural information such as a crystal structure. Further objects of the invention will become apparent to the person of routine skill in the art in view of the following detailed description of the invention.

This object and yet further objects are achieved inter alia by a method for the identification of suitable fragmentation sites in a reporter protein, and related thereto, recombinant DNA sequences and, encoded thereby, first and complementary second subdomains of a reporter protein, host cell lines transformed with said recombinant DNA sequences, a kit of parts comprising DNA-based expression vectors, a method for detecting an interaction between proteins, a use of random circular permutation and a use of a host cell line allowing for homologous recombination according to the independent claims.

Most biological processes are controlled by protein-protein interactions and split-protein sensors have become one of the few available tools for the characterization and identification of protein-protein interactions in living cells. Here we introduce a generally applicable combinatorial approach for the generation of new split-protein sensors and apply it to the (β/α)₈-barrel enzyme N-(5'-phosphoribosyl)-anthranilate isomerase Trp1p from *Saccharomyces cerevisiae* (cf. Braus, G.H., Luger, K., Paravicini, G., Schmidheini, T., Kirschner, K., and Hutter, R. (1988), J Biol Chem 263, 7868-7875). These so-called split-Trp protein sensors are capable of monitoring the interactions of pairs of cytosolic and membrane proteins. One of the selected split-Trp pairs (⁴⁴Nₜᵣₚ and ⁴⁴Cₜᵣₚ) was chosen by means of an example and successfully applied to monitor protein-protein interactions both at the membrane as well as in the cytosol of yeast. Its selected fragmentation site would not have been easily predicted by theoretical considerations, thus underlining the power of the evolutionary approach according to the invention. The direct read-out through complementation of tryptophan auxotrophy qualifies the split-Trp system for high-throughput applications in yeast and bacteria. Of course, appropriately engineered trp1-deficient host strains are required for such assays, which are however either readily available or easily to be made by the person of routine skill in the art. In addition, the introduced combinatorial approach allows for generating split-protein sensors of almost any reporter protein, thereby yielding tailor-made sensors for different applications.

Trp1p is a relatively small (25 kD), monomeric protein that catalyzes the isomerization of N-(5'-phosphoribosyl)-anthranilate in the biosynthesis of tryptophan (cf. Eberhard, M., Tsai-Pflugfelder, M., Bolewska, K., Hommel, U., and Kirschner, K. (1995), Biochemistry 34, 5419-5428). The DNA coding sequence of Saccharomyces cerevisiae is given in SEQ ID NO: 1, the corresponding amino acid sequence is given in SEQ ID NO: 2. Creating a pair of Trp1p fragments (split-Trp) that only reconstitute the enzymatic activity when linked to interacting proteins allows monitoring this protein interaction through a simple growth assay: otherwise *trp1* yeast strains expressing such a split-Trp fusion pair would not be able to grow on medium lacking tryptophan. As many different *trp1* strains exist, the interaction assay could be applied immediately in different genetic backgrounds, adding a further attractive feature to a split-Trp sensor. Trp1p is a well-studied member of the prominent class of proteins that fold into a (β/α)₈-barrel, which is the most commonly occurring fold among enzymes. The herein presented approach of identifying suitable fragmentation sites in a reporter protein is thus very broadly applicable. This folding motive has been previously subjected to circular permutation and has been expressed as two separate fragments that spontaneously associate into a functional enzyme (cf. Luger, K., Hommel, U., Herold, M., Hofsteenge, J., and Kirschner, K. (1989), Science 243, 206-210; Eder, J., and Kirschner, K. (1992), Biochemistry 31, 3617-3625). Furthermore, it has been proposed that the (β/α)₈-barrel evolved by tandem duplication from a (β/α)₄-domain (cf. Hocker, B., Schmidt, S., and Sterner, R. (2002), FEBS Lett 510, 133-135). In addition to any practical applications it would therefore add to our understanding where the (β/α)₈-barrel can be split into two fragments that, in contrast to previously described pairs of fragments, reconstitute quasi-native Trp1p only when fused to interacting proteins.

As used herein, a "reporter protein" is understood as a protein or peptide, which possesses a unique activity in vivo and/or in vitro, and which produces a signal that allows the active protein to be easily discernable even within a complex mixture of other proteins or peptides, especially in vivo. Reporter proteins as understood herein are e.g. (i) proteins which are essentially involved in the biosynthetic pathway of formation of an amino acid or an other essential metabolite that is crucial for the organism to survive on medium lacking the respective amino acid or metabolite; or (ii) proteins which are detectable by a characteristic color assay when, preferably in vivo; etc.

As used herein, a "suitable fragmentation site" is understood as an especially randomly chosen position in the amino acid chain (and/or the corresponding gene sequence, respectively), at which a given reporter protein is fragmented into a first subdomain and a complementary second subdomain (and/or the corresponding first subsequence and the complementary second subsequence, respectively), wherein the fragmentation site is suitable in the sense of the present invention, when it fulfils the following demands: (i) to yield two fragments that efficiently fold into quasi-native protein only when fused to two interacting proteins; (ii) not to significantly impair the activity of a reconstituted protein by bringing the two fragments into close proximity especially in vivo; (iii) to yield soluble protein fragments that are not readily degraded in vivo.

As used herein, the term "detectable", especially "detectable when active" is understood as follows. Detection in the sense of the present invention includes any direct or indirect method of testing for the presence of a reporter protein, especially when reconstituted by fragments thereof, e.g. by chemical, physical, or visual means. Most preferably, detection is performed by a color assay, e.g. fluorescence, chemiluminescence or the like, (in vivo and/or in vitro) and/or a growth assay (in vivo).

As used herein, a "first subdomain" and a "complementary second subdomain" of a reporter protein are understood as follows. A first subdomain represents a first successional part (either an N-terminal-, C-terminal-, integral part or even a part involving both the N-terminal- and the C-terminal part) of a native reporter protein. A complementary second subdomain represents a complementary second part (either an N-terminal, C-terminal, integral part or even a part involving both the N-terminal- and the C-terminal part). The first subdomain and the complementary second subdomain essentially resemble the wild-type sequence, when viewed together, wherein overlapping sequences between both subdomains, that are present in both the first subdomain and the complementary second subdomain can be tolerated as long as the activity of the enzyme is not significantly negatively affected. Moreover, minor deletions, additions or other alterations to the overall sequence can be tolerated, especially at the N-terminus or the C-terminus, as long as the activity of the reporter protein, either as a whole or when reconstituted by its fragments, is not significantly negatively affected.

As used herein, a "first subsequence" and a "complementary second subsequence" are understood as gene sequences encoding for the above-mentioned first subdomain and complementary second subdomain.

As used herein, a "color assay" is understood as a manually or device-supported detection of a change in optical appearance of a sample comprising the reporter protein, or a reporter protein reconstituted by its fragments, incl. color developments as well in the visible as in the invisible spectrum. Color assays are especially preferred, that can be qualitatively detected by the unaided eye e.g. by coloration of living cells in vivo (colonies on a plate or the like), and that can be additionally quantified in an in vitro assay, e.g. for determining the intensity of an interaction between two proteins.

As used herein, a "growth assay" is understood as an assay, that allows for the growth of a cell, e.g. a colony on a plate, when the reporter protein is present or actively resembled by its fragments, and wherein cells fail to grow, when the reporter protein is not present or actively resembled by its fragments. Most preferably, the growth assay suchlike allows for a simple visual selection of positives.

As used herein, "stringent conditions" for hybridization of DNA are understood as follows. Given a specific DNA sequence, a person of skill in the art would not expect substantial variation among species within the claimed genus due to hybridization under such conditions, thus expecting structurally similar DNA.

The method according to the invention for the identification of suitable fragmentation sites in a reporter protein, wherein the reporter protein is detectable when active, comprises the steps of:
(a) providing a DNA sequence encoding for said reporter protein;
(b) creating a library based on the DNA sequence as defined in (a),
   - wherein each individual of said library comprises a randomly created first subsequence of the DNA sequence as defined in (a), encoding for a first subdomain of said reporter protein, and
   - wherein each individual of said library comprises a randomly created complementary second subsequence of the DNA sequence as defined in (a), encoding for a complementary second subdomain of said reporter protein;
(c) screening and/or selection for restoration of detectable activity of said reporter protein, when said first subdomain and said complementary second subdomain are brought into close proximity;
(d) identifying said first subdomain and/or said first subsequence; and said complementary second subdomain and/or said complementary second subsequence, that lead to restoration of detectable activity of said reporter protein.

By using a combinatorial library approach, comprising randomly created first subsequences and randomly created complementary second subsequences, the drawbacks of rational design of split-protein sensors are overcome. Most advantageously, even fragmentation sites of proteins encoded by said subsequences may thereby be identified, which would have never been readily predicted by any rational approach. First subsequences and complementary subsequences are ideally suitable in the context of the present invention, when reconstitution of activity of the corresponding reporter protein only occurs to a significant extent at all, when both corresponding subdomains are forced into close spatial proximity, but do not self-assemble in order to reconstitute a detectable amount of an active reporter protein.

DNA sequences of suitable reporter proteins are readily available to the person of routine skill in the art (step (a)), e.g. from the National Center for Biotechnology Information (NCBI), National Library of Medicine, Building 38A, Bethesda, MD 20894. Genes encoding for reporter proteins may then be amplified e.g. from a suitable host cell by PCR using standard techniques and primers suitably designed based on the known DNA sequence (vide supra), or the gene encoding for a reporter protein may be completely built up from suitably designed oligonucleotides de novo.

DNA manipulating techniques that may be used in step (b) for the creation of a library based on said DNA sequence are readily apparent to the person of routine skill in the art, either. In short, N- and C-terminal domains of the wild-type reporter protein are amplified separately from a suitable source of DNA by standard PCR techniques, and are subsequently recombined using standard overlap extension PCR techniques in order to recombine and thereby re-arrange the wild-type gene, preferably now containing the N- and C-termini of the wild-type gene connected with each other and as an internal part of the sequence, and preferably comprising a unique restriction site at the wild-type N- and C-termini. At the same time, suitable restriction sites may be designed at the newly created N- and C-termini in order to allow for efficient subsequent cloning steps; most preferably, the restriction site is designed for the same restriction enzyme at both the N- and C-terminus. Most preferably, the re-arranged DNA construct is inserted into a high-copy plasmid, the plasmid amplified by standard techniques, and the re-arranged DNA of interest is thereafter cut out of the high-copy plasmid using the restriction sites at the newly created N- and C-termini. The rearranged gene is then incubated with a ligase to yield dimerized, oligomerized and circularized DNA construct. Afterwards, these constructs are digested e.g. with a suitable, random-cut DNAse, and fragments corresponding to the wild-type length are preferably thereafter treated with ligase and polymerase to repair nicks, gaps and to flush the ends of the fragments of the reporter protein. Afterwards, the DNA fragments corresponding to the wild-type length of the reporter protein's gene are isolated e.g. by standard agarose gel electrophoresis procedures. The resulting fragments are preferably blunt-end cloned into a suitable expression vector, which was cleaved at a unique restriction site (preferably blunt-end). The expression vector is especially designed by standard DNA manipulation techniques to provide a construct after blunt-end cloning, in which one of the artificially generated new N- and C-termini is under, the control of a promoter sequence and especially fused to a gene encoding for a tag sequence and a gene encoding for first peptide or protein C1, each preferably via a linker sequence. Moreover, the other terminus, respectively, is especially fused to a gene encoding for a preferably different tag sequence and gene encoding for a second peptide or protein C2. Peptides or proteins C1 and C2 are thereby known to interact with each other in vivo, and may e.g. be leucine zippers. The tag sequences may afterwards advantageously be used for the control of correct expression and stability of fusion proteins. After transformation and amplification in a suitable host such as e.g. E.coli XL1Blue to a typical library size of about 10⁴ to 10⁵ independent clones, the vector is linearized at a restriction site at the wild-type N- and C-termini, and an oligonucleotide is inserted into the resulting gap, which is specifically designed to integrate a terminator for the first domain of said reporter protein and a promoter sequence for the second domain of said reporter protein, by homologous recombination in a suitable host such as yeast according to standard procedures. The oligonucleotide is designed and constructed by standard PCR techniques to provide flanking regions both at the 5' and 3' ends of e.g. about 50bp with the gene of the reporter protein in order to allow for successful homologous recombination. Suchlike, the selection of clones possessing fragmentation sites at or nearby the wild-type N- and C-termini can be suppressed. For selecting thereafter, a marker gene is also provided by the oligonucleotide, e.g. encoding for a protein involved in antibiotic resistance. Successful homologous recombination may thus be easily observed by growth in the presence of the respective antibiotic.

Step (c) is preferably carried out by growing the respective transformants of the library on medium which e.g. lacks a nutrient, e.g. an amino acid, or which provides a substrate for a color reaction. Thus, preferably a growth assay or a color assay is performed, thereby allowing for easy selection of those transformants which lead to a restoration of activity of the reporter protein, which is e.g. essentially involved in the synthesis of said nutrient, e.g. said amino acid, or in said color reaction. Step (c) especially involves the elimination of false positives, i.e. first subdomains and complementary second subdomains, that reconstitute an active reporter enzyme by self-reassembling, i.e. without the need of an outer influence forcing the two domains into close spatial proximity. This can be done e.g. by fusing the respective first and second subdomains of the reporter protein to first and second peptides or proteins, that do not interact with each other, and/or by testing the respective first and second subdomains without any first and second peptides fused thereto at all, and/or by testing constructs lacking the first or the second subdomain, respectively. These assays can be performed by techniques commonly known in the art of e.g. two-hybrid assays.

Identification of suitable subdomains and subsequences, i.e. suitable fragementation sites, can be performed by common DNA- and/or protein sequencing techniques.

According to a preferred embodiment, the reporter protein is detectable in vivo and/or in vitro, both as full length protein and when actively resembled by a first subdomain and a complementary second subdomain, by a means chosen from the group consisting of color assays and growth assays.

Growth assays provide the advantage of a selection step, i.e. only positives grow under the chosen conditions, thus eliminating the need of further screening all individuals of the library. Exemplarily, only positives that comprise a suitable combination of first subdomain and complementary second subdomain grow as colonies on nutrition-specific plates. Color assays, moreover, can be individually designed depending on the specific reporter protein, when this reporter protein is involved naturally in or artificially usable for a color-developing reaction. In some cases, a substrate for such a reporter protein may be incorporated into the growth medium, e.g. the plate, whereupon colored colonies appear due to reconstitution of an active reporter protein by a first subdomain and a complementary second subdomain in vivo. Quanification of such an in vivo color assay may be optionally performed with samples obtained from such colonies. The general procedure of growth assays, color assays and subsequent quantification of the color assay are known in principle from the classical two-hybrid system, cf. eg. US 5,667,973.

In an especially preferred embodiment, individuals of the library as defined in (b) are either prokaryotic or eukaryotic host cells, comprising:
- both said first subsequence and said complementary second subsequence in one and the same expression vector, suitable for (co-)expression of said first subsequence and said complementary second subsequence in vivo; or
- said first subsequence in a first expression vector suitable for (co-)expression of said first subsequence, and said complementary second subsequence in a second expression vector suitable for (co-)expression of said complementary second subsequence.

In vivo assays are at least in the first step preferred, e.g. as a growth assay as outlined above. Thus, prokaryotic or eukaryotic host cells are provided, that are manipulated suchlike to allow for the (co-)expression of both the first and the complementary second subdomain of the reporter protein. Depending on the specific application, both subdomains may of course be encoded by one and the same, or by separate vectors. In most cases, encoding by one and the same vector will be favourable. A vast amount of suitable expression vectors for use as a basis in this respect are available to the person of routine skill in the art, e.g. the pRS316-based yeast expression vector (cf. Sikorski, R.S., and Hieter, P. (1989), Genetics 122, 19-27). It is especially preferred that the screening for restoration of detectable activity of said reporter protein, when said first subdomain and said complementary second subdomain are brought into close proximity as defined in (c), comprises the following steps:
- creating a first fusion subsequence comprising the first subsequence of said reporter protein as defined in (b), fused to an oligonucleotide encoding for a first protein or peptide,
- creating a second fusion subsequence comprising the complementary second subsequence of said reporter protein as defined in (b), fused to an oligonucleotide encoding for a second protein or peptide,
wherein said first protein or peptide and said second protein or peptide are known to interact.

By creating said first fusion sequence and said second fusion subsequence, the first subdomain and the complementary second subdomain are forced into close spatial proximity, thus allowing for a screening for restoration of activity of the reporter protein, when the subdomains are forced into close proximity. Preferably, said first protein or peptide and said second protein or peptide are chosen to be robust and relatively small proteins or peptides; especially preferred in the context of the invention are leucine zippers, most preferably leucine zippers which associate to an anti-parallel coiled coil (interacting proteins fused to 3'-terminus of the first subdomain and the 5'-terminus of the second subdomain, or vice versa, respectively). However, for specific embodiments, a parallel orientation may be preferred, e.g. for testing membrane proteins which most commonly exhibit both the N- and the C-terminus to one and the same site.

According to a further embodiment said first fusion subsequence and said second subsequence are created by blunt end ligation.

Blunt end ligation is the method of choice for the construction of said fusion subsequences, as due to the evolutionary, random approach of library generation no predictable, specific sticky-end ligation can be performed. Although blunt-end ligation leads to the creation of statistical amounts of ligation products which are out of the reading frame, this approach still proved sufficiently efficient for the identification of suitable fragmentation sites according to the invention.

Moreover, in another especially preferred embodiment said first fusion subsequence and said second fusion subsequence each comprise
- a linker sequence in between said first subsequence (or said second subsequence, respectively) and said oligonucleotide encoding for a first protein or peptide (or said oligonucleotide encoding for a second protein or peptide, respectively);
- at least one tag that allows for verification of the transcription of said first fusion subsequence and said second fusion subsequence.

Linker sequences commonly prove useful in the art of construction of fusion proteins in order to both allow for proper folding of both components of the fusion protein individually or cooperatively, and/or to achieve sufficient spatial integrity of both components of the fusion protein.

The use of tag sequences that allow for the detection of transcription of a gene sequence is also routinely applied in the art. In the context of the present invention, tag sequences may be applied to any of the N- and C-terminus of the first subdomain and/or the N- and C-terminus of the complementary second subdomain. It is especially preferred to provide differently recognizable tag sequences both at the N- and the C-termini of each transcription product. Commonly applied tags are e.g. the HA tag, the flag tag or the like. Detection of correct expression of these tags, and thereby of the fusion protein(s), may be performed e.g. by Western-blotting according to routine procedures.

According to an especially preferred embodiment, an oligonucleotide is inserted by homologous recombination in between said first subsequence and said second subsequence, encoding for:
- a transcription terminating sequence for terminating transcription of said first or said second subsequence;
- a transcription promoting sequence for initiating transcription of said second or said first subsequence, respectively;
- a marker sequence allowing for control of successful homologous recombination.

An especially advantageous way of carrying out the present invention is to simply initially provide said first and said second subsequence continuously, preferably rearranged, and thereafter to separate them by introducing a transcription terminating sequence succeeding the first subsequence, and a transcription promoting sequence preceeding the second subsequence. Thereby, separate expression is secured of both the first subdomain and the complementary second subdomain, or their fusion domains, respectively. This goal may be especially advantageously achieved by homologous recombination at a predefined site in between said first and said second subsequence (c.f. Oldenburg, K.R., Vo, K.T., Michaelis, S., and Paddon, C. (1997), Nucleic Acids Res 25, 451-452).

In order to eliminate the otherwise high risk of isolating subdomains, that are fragmented at fragmentation sites nearby the N- and C-termini of the wild-type reporter protein, it is especially preferred to not provide the DNA sequence of said reporter protein according to step (a), vide supra, in its wild-type configuration, but rather already with the wild-type N- and C-termini connected with each other and being an internal part of the DNA sequence of said DNA sequence. Thereby, artificial new N- and C-termini are created in the starting material. Most preferably, a unique restriction site RE2 is introduced in between the wild-type N- and C-terminus. A further restriction site RE1 is advantageously introduced at the new artificial N- and C-terminus of the DNA sequence of said reporter protein according to step (a), allowing for easy and convenient cloning and construction of libraries according to step (b), vide supra. Due to the unique restriction site RE2, homologous recombination in a suitable host cell can be performed in between the wild-type N- and C-terminus of the reporter protein. Due to the necessary overlap for successful homologous recombination, isolation of subdomains with fragmentation sites at or nearby the wild-type N- and C-terminus is suppressed. Most preferably, the oligonucleotide used for homologous recombination comprises a selection marker such as e.g. a gene involved in antibiotic resistance in order to check for successful homologous recombination.

Thus, in a further embodiment, the method comprises the steps of:
- creating fragmentation sites in TRP1 using gene cleavage with a unique restriction enzyme RE1 and circularization;
- isolating fragments corresponding to the wild-type length;
- subcloning using blunt ends preferably into a pRS316 based yeast expression vector under the control of a copper promoter (pCUB1) and transforming into E. coli, preferably XL1Blue;
- recombining and amplifying homologues with a unique restriction site RE2, preferably AvrII, introduced between the original N- and C-termini to allow subsequent linerization of the vector;
- locating two leucine zippers in the plasmid at the 3'- and the 5'-ends of the newly generated N- and C-termini, the zippers being positive and negative charged helices to allow heterodimerization, preferably each heterodimer containing a buried asparagine residue in a position to force antiparallel orientation of the zippers.

The invention further relates to a recombinant DNA sequence for use in securing expression in a prokaryotic or eukaryotic host cell of a polypeptide product having the primary structural conformation of a first subdomain of a reporter protein or a complementary second subdomain of a reporter protein, wherein detectable activity of said reporter protein is restored, when said first subdomain and said complementary second subdomain are brought into close proximity, and wherein said first and said complementary second subdomain are not subdomains of one of the group of proteins consisting of transcriptional activators, ubiquitin, dihydrofolate reductase, β-lactamase, green fluorescent protein and closely related variants such as e.g. ECFP, EGFP or the like, β-galactosidase, inteins, cAMP cyclase, glycinamide ribonucleotide transformylase, aminoglycoside

In the above-mentioned and herewith disclaimed DNA sequences, suitable fragmentation sites for split-protein sensors were already identified by rational design (cf. e.g. Methods Enzymology 238, Michnick et al. 2000). However, the present invention now opens up for the first time the possibility to identify suitable fragmentation sites in any other DNA sequence encoding for a reporter protein by a random library approach, too. Providing this tool to the person of routine skill in the art by the method disclosed herein, suitable fragmentation sites may be now identified with relative ease.

A DNA sequence according to the invention encodes for a subdomain of a (β/α)₈-barrel enzyme, such as Trp1p.

According to the invention said DNA sequence is selected from the group consisting of:
(a) the DNA sequences set out in Table 1 and their complementary strands;
b) DNA sequences which, but for the degeneracy of the genetic code, would hybridize to the DNA sequences defined in (a) and which sequences code for a polypeptide having the same amino acid sequence.

The above-mentioned DNA sequences encode for the split-Trp sensors split-Trp⁴⁴ (i.e. ⁴⁴Nₜᵣₚ and ⁴⁴Cₜᵣₚ), split-Trp⁵³ (i.e. ⁵³Nₜᵣₚ and ⁵³Cₜᵣₚ), split-Trp¹⁸⁷ (i.e. ¹⁸⁷Nₜᵣₚ and ¹⁸⁷Cₜᵣₚ), split-Trp^{204b} (i.e. ^{204b}Nₜᵣₚ and ^{204b}Cₜᵣₚ), which proved to be valuable tools as split-protein sensors (numbering according to the fragmentation site, given as the last amino acid of the N-terminal subdomain). Especially split-Trp44 was successfully applied herein to demonstrate the interaction of membrane proteins.

The DNA- and amino acid sequences of the above-mentioned split-Trp sensors are given in the attached sequenced listing as follows (SEQ ID No 7, 8, 9, 10 not according to the invention):

**Table 1:**

| | |
|---|---|
| SEQ ID NO: 3 | ⁴⁴Nₜᵣₚ (DNA sequence); |
| SEQ ID NO: 4 | ⁴⁴Nₜᵣₚ (amino acid sequence); |
| SEQ ID NO: 5 | ⁴⁴Cₜᵣₚ (DNA sequence); |
| SEQ ID NO: 6 | ⁴⁴Cₜᵣₚ (amino acid sequence); |
| SEQ ID NO: 7 | ⁵³Nₜᵣₚ (DNA sequence); |
| SEQ ID NO: 8 | ⁵³Nₜᵣₚ (amino acid sequence); |
| SEQ ID NO: 9 | ⁵³Cₜᵣₚ (DNA sequence); |
| SEQ ID NO: 10 | ⁵³Cₜᵣₚ (amino acid sequence); |
| SEQ ID NO: 11 | ¹⁸⁷Nₜᵣₚ (DNA sequence); |
| SEQ ID NO: 12 | ¹⁸⁷Nₜᵣₚ (amino acid sequence); |
| SEQ ID NO: 13 | ¹⁸⁷Cₜᵣₚ (DNA sequence); |
| SEQ ID NO: 14 | ¹⁸⁷Cₜᵣₚ (amino acid sequence); |
| SEQ ID NO: 15 | ^{204b}Nₜᵣₚ (DNA sequence); |
| SEQ ID NO: 16 | ^{204b}Nₜᵣₚ (amino acid sequence); |
| SEQ ID NO: 17 | ^{204b}Cₜᵣₚ (DNA sequence); |
| SEQ ID NO: 18 | ^{204b}Cₜᵣₚ (amino acid sequence); |

In preferred embodiments according to the present invention, said DNA sequences are used in securing expression in a prokaryotic or eukaryotic host cell of a polypeptide fusion product. Such securing of expression may be achieved by any means routinely applied by the person of routine skill in the art, comprising e.g. incorporation of said DNA sequences into suitable expression vectors or integration of said DNA sequences into the genome of said host.

The invention further relates to a first subdomain of a reporter protein or a complementary second subdomain of a reporter protein, wherein detectable activity of said reporter protein is restored, when said first subdomain and said complementary second subdomain are brought into close proximity, and wherein said first and said complementary second subdomain are not subdomains of one of the group of proteins consisting of transcriptional activators, ubiquitin, dihydrofolate reductase, β-lactamase, green fluorescent protein and closely related variants such as e.g. ECFP, EGFP or the like, β-galactosidase, inteins, cAMP cyclase, glycinamide ribonucleotide transformylase, aminoglycoside phosphotransferase, hygromycin B phosphotransferase, luciferase.

In the above-mentioned and herewith disclaimed proteins, suitable fragmentation sites for split-protein sensors were already identified by rational design. However, the present invention now opens up for the first time the possibility to identify suitable fragmentation sites in any other reporter protein by a random library approach, too. Providing this tool to the person of routine skill in the art by the method disclosed herein, suitable fragmentation sites may be now identified with relative ease.

According to the invention, a first subdomain of a reporter protein or a complementary second subdomain of a reporter protein are produced by a method of culturing a host transformed with a recombinant DNA sequence as outlined above, wherein said molecules further comprises an expression control sequence, said expression control sequence being operatively linked to said molecule. Said expression control sequences comprise especially those which are commonly referred to as tags which are recognizable e.g. by Western-blotting procedures routinely applied in the art.

The invention further relates to a fusion protein comprising a first subdomain of a reporter protein or a complementary second subdomain of a reporter protein as outlined above, and a further peptide or protein connected thereto in a naturally not occurring combination. By creating such artificial fusion proteins, said further protein of peptide may then be tested for interaction with e.g. a specifically chosen counterpart or against a library of possible counterparts. Moreover, library-library screening assays may also be applied, e.g. genome-wide library screenings as e.g. already performed in the art of traditional two-hybrid assay.

The invention further relates to a prokaryotic or eukaryotic host cell line, transformed with recombinant DNA sequences as outlined above.

Said prokaryotic or eukaryotic host cell lines are preferably E. coli or yeast strains. For cloning and storage purposes, mostly E. coli strains such as XL1Blue will be chosen. For the method of identification of suitable fragmentation sites according to the invention, especially involving the step of homologous recombination, a yeast strain may be chosen such as e.g. Saccharomyces cerevisiae, e.g. EGY48, and Schizosaccharomyces pombe. The choice of a suitable host cell line is routinely performed by the person of skill in the art, depending on the specific purpose; such host cell lines are commonly available.

The invention is further related to a kit of parts, comprising a first and a second DNA-based expression vector, wherein
- said first expression vector contains an expression cassette encoding for a polypeptide product having at least a substantial part of the primary structural confirmation of a first subdomain of a reporter protein; and
- said second expression vector contains an expression cassette encoding for a polypeptide product having at least a substantial part of the primary structural confirmation of a complementary second subdomain of a reporter protein; and
wherein detectable activity of said reporter protein is restored, when said first subdomain and said complementary second subdomain are brought into close proximity, and wherein said first and said complementary second subdomain are not subdomains of one the group of proteins consisting of transcriptional activators, ubiquitin, dihydrofolate reductase, β-lactamase, green fluorescent protein and closely related variants such as e.g. ECFP, EGFP or the like, β-galactosidase, inteins, cAMP cyclase, glycinamide ribonucleotide transformylase, aminoglycoside phosphotransferase, hygromycin B phosphotransferase, luciferase.

According to a further especially preferred embodiment, such a kit of parts further comprising a suitable prokaryotic or eukaryotic host cell line for expression of said first and second expression vector.

Having provided by the present invention a tool for identifying novel fragmentation sites in reporter proteins, another major aspect of the present invention is related to a method for detecting an interaction between a first test peptide or protein or a fragment thereof, and a second test peptide or protein or a fragment thereof, the method comprising the steps of:
- providing recombinant DNA sequences as outlined above for use in securing expression of a first subdomain of a reporter protein and a complementary second subdomain of a reporter protein;
- fusing an oligonucleotide or a gene encoding for a first test peptide or protein to the DNA sequence encoding for said first subdomain of the reporter protein, thereby creating a first DNA fusion sequence encoding for a fusion protein comprising said first subdomain of the reporter protein and said first test peptide or protein;
- fusing an oligonucleotide or a gene encoding for a second test peptide or protein to the DNA sequence encoding for said complementary second subdomain of the reporter protein, thereby creating a second DNA fusion sequence encoding for a fusion protein comprising said complementary second subdomain of the reporter protein and said second test peptide or protein;
- (co-)expressing said fusion protein comprising said first subdomain of the reporter protein and said first test peptide or protein, and said fusion protein comprising said second complementary subdomain of the reporter protein and said second test peptide or protein in a suitable prokaryotic or eukaryotic host cell;
- screening and/or selecting for restoration of detectable activity of said reporter protein.

Utilizing split-protein sensors with subdomains identified by a method according to the invention, interaction of said first test peptide and said second test peptide may be identified. Given the tool of identifying suitable fragmentation sites in virtually any reporter protein, the person of routine skill in the art is no more hampered by the limitations of the existing, rationally designed split-protein systems to specific cellular compartments, but rather may now choose a reporter protein depending on his specific test purpose.

In the most preferred embodiment, a library of oligonucleotides or DNA encoding for a set of first test peptides or proteins and/or a library of oligonucleotides or DNA encoding for a set of second test peptides or proteins are fused to said first subdomain of said reporter protein and/or said complementary second subdomain of said reporter protein, respectively.

According to an especially preferred embodiment of the present invention, the interaction between a first test peptide or protein or a fragment thereof and a second test peptide or protein or fragment thereof is mediated by a chemical inducer of dimerization, which binds either covalently or non-covalently to both said test peptides or proteins or fragments thereof.

Comparable systems are commonly referred to in the literature as three-hybrid systems. Chemical inducers of dimerization (CIDs) have been first described by Schreiber and Crabtree (c.f. Spencer D.M, Wandless T.J, Schreiber S.L, and Crabtree G.R (1993), Science 262, 1019-1024). CIDs are cell-permeable molecules that can simultaneously form a covalent- or non-covalent interaction with two different proteins or peptides, thereby inducing their dimerization. Using split-protein sensors according to the present invention, e.g. robust drug and/or drug target screening assays may easily be established. Towards this aim, e.g. Nₜᵣₚ may be fused to a protein library and Cₜᵣₚ to an O(6)-alkylguanine-DNA alkyltransferase (AGT), e.g. human AGT (hAGT). A substrate for hAGT, e.g. Benzylguanine, may be easily covalently linked to a multitude of small molecules (hypothetical drugs), thus allowing for an efficient screening for cellular targets contained in said protein library that react or associate with the corresponding drug.

Moreover, the invention is related to a method for detecting the interruption of an interaction between a first test peptide or protein or a fragment thereof, and a second test peptide or protein or a fragment thereof, the method comprising the steps of:
- providing recombinant DNA sequences according to one of claims 11 to 12 for use in securing expression of a first subdomain of a reporter protein and a complementary second subdomain of a reporter protein;
- fusing an oligonucleotide or a gene encoding for a first test peptide or protein to the DNA sequence encoding for said first subdomain of the reporter protein, thereby creating a first DNA fusion sequence encoding for a fusion protein comprising said first subdomain of the reporter protein and said first test peptide or protein;
- fusing an oligonucleotide or a gene encoding for a second test peptide or protein to the DNA sequence encoding for said complementary second subdomain of the reporter protein, thereby creating a second DNA fusion sequence encoding for a fusion protein comprising said complementary second subdomain of the reporter protein and said second test peptide or protein;
- (co-)expressing said fusion protein comprising said first subdomain of the reporter protein and said first test peptide or protein, and said fusion protein comprising said second complementary subdomain of the reporter protein and said second test peptide or protein in a suitable prokaryotic or eukaryotic host cell;
- screening and/or selecting for interruption of interaction of said first subdomain and said second subdomain under the influence of one or more test agents.

Comparable systems are commonly referred to in the literature as reverse two-hybrid systems (or split-protein systems, respectively). Exemplarily, 5-fluoroanthranilic acid (FAA) is metabolized in vivo into a toxic product by the tryptophan biosynthetic enzymes. Applying the split-Trp sensors according to the invention, the disruption of protein-protein interaction leading to the spatial separation of the Trp1p fragments (and thus inactivity of the reporter protein) can therefore be linked to the survival of the cells on medium containing FAA. By means of example, libraries of small molecules may be screened for their ability to interact with a pair of fusion proteins. Selection of proteins or peptides that disrupt an interaction can be done by co-expressing two interacting proteins with a random protein or peptide library e.g. on plates containing FAA. The reverse split-Trp sensors may also advantageously be used to determine the binding region of a protein. A random library of the protein carrying mutations is co-expressed with its binding partner on plates containing FAA. Only cells that express a library member with mutations in or affecting the binding region, thus disrupting the interaction of the two proteins, will be able to grow in the presence of FAA.

Another aspect of the present invention is related to a use of random circular permutation of a gene and/or the expressed polypeptide derived thereof for the identification of fragmentation sites in a reporter protein for use in a split-protein sensor. To date, random circular permutation has not been used for the identification of such suitable fragmentation sites for separately expressed subdomains, but rather for the identification of proteins of at least approximately wild-type length, but with artificially new N- and C-termini, and with the wild-type N- and C-termini being connected to each other and being an internal part of the sequence. However, this approach now surprisingly proved to be an outstandingly valuable tool for the evolutionary, combinatorial approach of identifying suitable fragmentation sites for subdomains to be expressed separately.

A further aspect of the present invention is related to a use of a host cell line that allows for homologous recombination of DNA for the generation of a recombinant DNA molecule that secures for expression of both a polypeptide product comprising a first subdomain of a reporter protein and a complementary second subdomain of a reporter protein from said recombinant DNA molecule. To date, homologous recombination has not been used for this purpose, but has now surprisingly found to be an outstandingly valuable tool for simply and conveniently securing for expression of a first subdomain and a complementary second subdomain of a reporter protein, in a method of one of claims 1-10.

### Detailed description of the invention

The invention will now be described in even more detail by means of an example and a specific embodiment, together with the accompanying figures; however, without the invention being limited thereto.

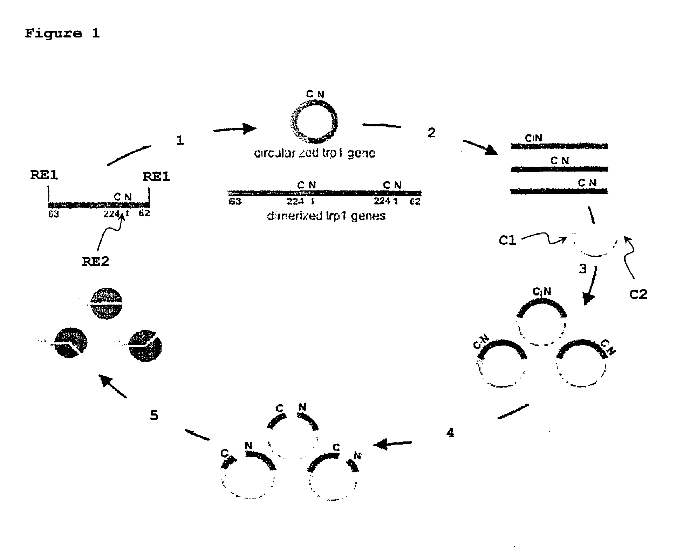

- Fig. 1:: Combinatorial approach towards the generation of split- Trp sensors. As a starting point, a rearranged copy of the TRP1 gene was used in which the original N- and C- termini of TRP1 were connected by a short linker encod- ing a unique restriction site RE2, here an AvrII site. For convenient subcloning, another restriction site RE1 was introduced at the artificially created new N- and C-termini, here a HindIII site. The linear fragment was incubated with T4 DNA ligase to circularize/oligomerize the gene (step 1). Treatment of the ligation mix with DNAseI resulted in randomly cut linear molecules and fragments corresponding to the size of TRP1 were iso- lated (step 2). Isolated fragments were cloned into a yeast expression vector containing two polypeptides (C1 and C2) that associate into an antiparallel-coiled coil (step 3). Homologous recombination in yeast cells was used to insert a terminator sequence and the P*_{GAL1}*- promoter between the original N- and C-termini (step 4). Co-expression of the two fragments and selection for complementation of tryptophan auxotrophy of yeast cells allowed the isolation of functional split-Trp pairs.

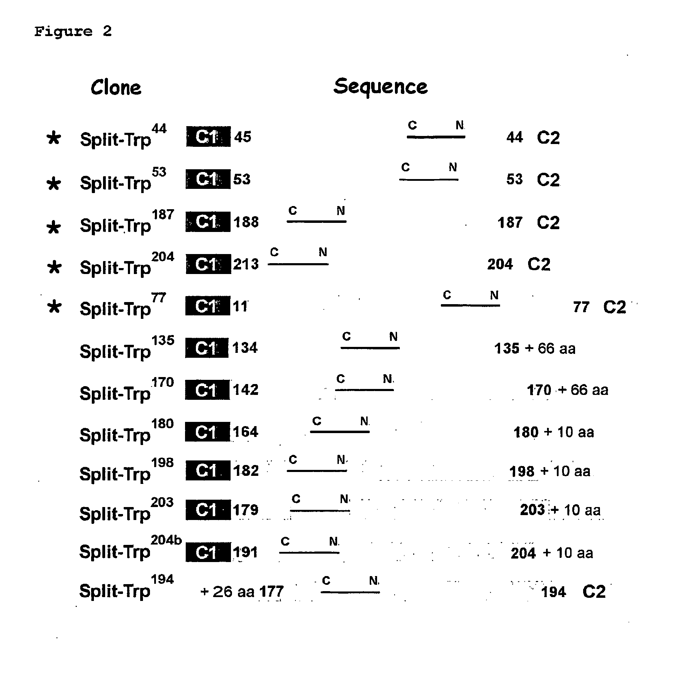

- Fig. 2:: Selected split-Trp protein pairs capable of complement- ing tryptophan auxotrophy in yeast. The clones are named after the last residue of each N-terminal frag- ment. C1 and C2 are the two polypeptides that associate into the anti-parallel coiled coil. Due to a shift in the reading frame in 5 of the twelve clones, C2 is re- placed by peptide of 10 or 66 amino acids, and C1 is replaced in one clone by a peptide of 26 residues. Five of the twelve analyzed clones lead to the expression of Trp1p fragments in which both fragments were fused in frame to the polypeptides C1 and C2 (marked with an as- terisk).

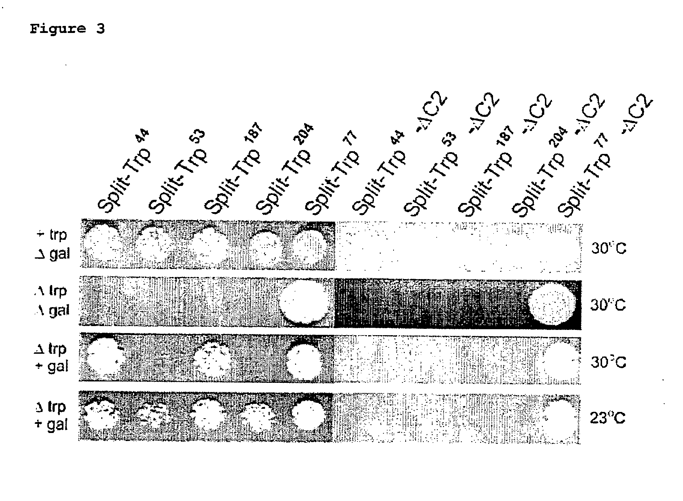

- Fig. 3:: Characterization of the selected split-Trp pairs that are marked with an asterisk in Fig. 2. Growth assays of yeast strains expressing split-Trp⁴⁴, split-Trp⁵³, split-Trp¹⁸⁷, split-Trp^{204b} or split-Trp⁷⁷ on selective plates (+/Δ trp: plates with tryptophan / lacking tryp- tophan, respectively; +/Δ gal: plates with galactose / lacking galactose). For control experiments, yeast strains expressing the split-Trp proteins in which the sequence encoding for C2 was deleted form the plasmid (split-Trp-ΔC2) were also investigated. One colony of yeast cells EGY48 expressing different split-Trp pro- tein pairs was resuspended in 1ml water and 5µl were spotted on medium with or without tryptophan and/or ga- lactose, but always containing copper at two different temperatures (30°C and 23°C). C1-Cₜᵣₚ is under control of the leaky P*_{CUP1}*-promoter and Nₜᵣₚ-C2 under the control of the P*_{GAL1}*-promoter. Images were taken after 8 days.

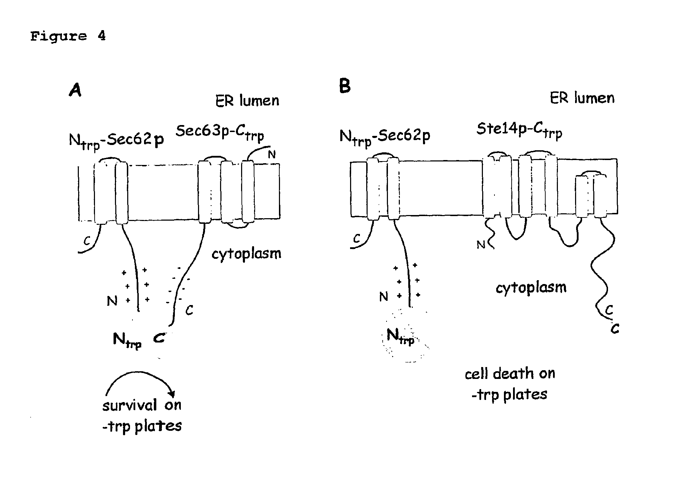

- Fig. 4:: Analysis of the interaction between Sec62p and Sec63p using the split-Trp system. Left: Nₜᵣₚ is fused to the N terminus of Sec62p and Cₜᵣₚ is fused to the C terminus of Sec63p, resulting in Nₜᵣₚ-Sec62p and Sec63p-Cₜᵣₚ, re- spectively. The linker between the cytosolic domains of Sec62p and Sec63p and the corresponding Trp1p fragments consists of six residues. The known interaction between the positively charged cytosolic N-terminal domain of Sec62p and the negatively charged C-terminal tail of Sec63p should lead to the reconstitution of active Trp1p and complementation of tryptophan auxotrophy. Right: Co-expression of Nₜᵣₚ-Sec62p with Ste14p-Cₜᵣₚ, a further membrane protein of the ER, which does not interact with Sec62p, should not lead to the formation of a functional Trp1p and the complementation of tryptophan auxotrophy.

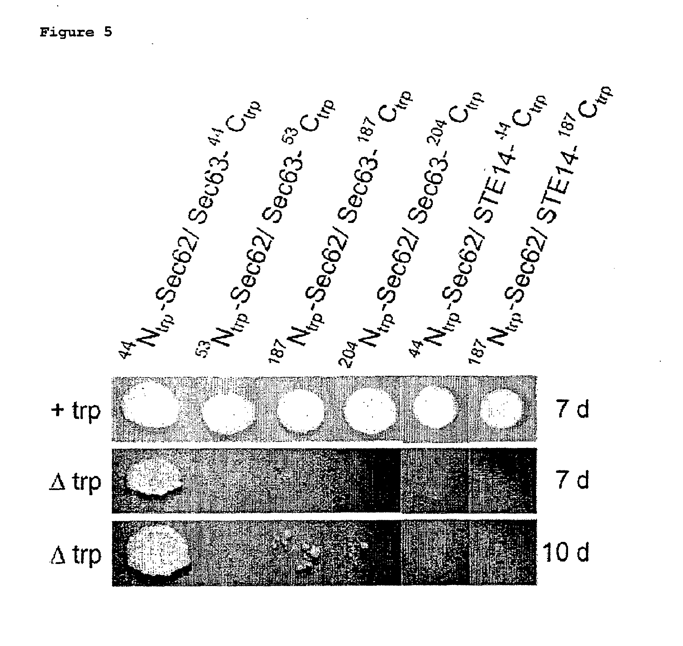

- Fig. 5:: Split-Trp interaction assay of Sec62p and Sec63p. A colony of EGY48 cells co-expressing Nₜᵣₚ-Sec62p with Sec63p-Cₜᵣₚ or Ste14p-Cₜᵣₚ was suspended in 1 ml water and 5 µl were spotted on copper containing medium with or without tryptophan. Cells co-expressing ⁴⁴Nₜᵣₚ- Sec62p/ Sec63p-⁴⁴Cₜᵣₚ complement tryptophan auxotrophy as indicated by their growth after 4 days at 23°C. Large colonies were visible after 7 days of incubation, whereas only small colonies were observed for cells ex- pressing ¹⁸⁷Nₜᵣₚ-Sec62p/ Sec63p-¹⁸⁷Cₜᵣₚ. No or only very small colonies were observed for cells co-expressing ⁵³Nₜᵣₚ-Sec62p/ Sec63p-⁵³Cₜᵣₚ or ^{204b}Nₜᵣₚ-Sec62p/ Sec63p- ^{204b}Cₜᵣₚ, respectively. No growth was observed for cells co-expressing ⁴⁴Nₜᵣₚ-Sec62p/ Ste14p-⁴⁴Cₜᵣₚ or ¹⁸⁷Nₜᵣₚ- Sec62p/ Ste14p-¹⁸⁷Cₜᵣₚ even after 10 days of incubation at 23°C.

DNA- and protein sequences SEQ ID NO: 1 to SEQ ID NO: 66, as given in the attached sequence listing, are given in the attached sequence listing, incl. all primers and oligonucleotides used for the construction of the vectors.

For any standard molecular biology and especially DNA- and protein manipulation protocols it is generally referred to Sambrook, J. et al., eds., MOLECULAR CLONING, A LABORATORY MANUAL, 2nd. edition, Cold Spring Harbor Laboratory Press, Cold Spring Harbor, N.Y. (1989); Ausubel, F. et al., eds., CURRENT PROTOCOLS IN MOLECULAR BIOLOGY, John H. Wiley & Sons, Inc. (1997); HYBRID HUNTER™ INSTRUCTION MANUAL, Invitrogen BV, Groningen, Neherlands (1999); Burke, D et al., METHODS IN YEAST GENETICS. A COLD SPRING HARBOR LABORATORY COURSE MANUAL, Cold Spring Harbor Laboratory Press (2000).

Yeast Media. Yeast complete medium containing adenine (YPAD) was used for cultures of *Saccharomyces cerevisiae* EGY48 and RSY529. Dropout media (YC) were used to select for the presence of pRS315- or pRS316-derived plasmids and for the complementation of tryptophan auxotrophy. Lacking amino acids or components in the resulting medium are indicated by the addition of their one-letter code to the YC- dropout medium. Selective YC-medium used to plate out the yeast cells after transformation by electroporation was supplemented with 1 M sorbitol. For the expression of proteins from the P*_{GAL1}*-promoter 2 % galactose and 0.5 % raffinose replaced glucose as carbon source in the YC-medium.
- YPAD:: 1 % yeast extract, 2 % peptone, 2 % dextrose, 100 mg/ l adenine, (2 % agar for plates)
- YC: 0.12 % yeast nitrogen base, 0.5 % ammonium sulfate, 1 % succinic acid, 2 % glucose, 0.6 % NaOH, 1.4 g/ l yeast synthetic dropout medium omitting histidine (H), leu- cine (L), tryptophan (W) and uracil (U), (2 % agar for plates),
-L: 0.05 g/ l histidine (H), 0.1 g/ l tryptophan (W), 0.1 g/ l uracil (U)
-U: 0.05 g/ l histidine (H), 0.1 g/ l leucine (L), 0.1 g/ l tryptophan (W)
-LU: 0.05 g/ l histidine (H), 0.1 g/ l tryptophan (W)
-LUW: 0.05 g/ l histidine (H),

Transformation of yeast cells. The transformation of *Saccharomyces cerevisiae* strains EGY48 or RSY529 with one or more plasmids was done using a standard protocol for transformation by electroporation. An overnight culture of EGY48 or RSY529 yeast cells in YPAD medium was diluted in 500 ml YPAD to an OD₆₀₀ of ∼0.3 and grown at 30°C and 260 rpm to an OD₆₀₀ of ∼1.4. The culture was harvested by centrifugation at 4300 rpm and washed with 500 ml and 250 ml ice-cold sterile water and with 30 ml ice-cold 1 M sorbitol. The pelleted cells were then resuspended in 300 - 500 µl 1 M sorbitol and either used directly for transformation or frozen in aliquots of 40 µl at -80°C. For the double transformation of two plasmids, competent cells were always prepared freshly. A total amount of 100 ng plasmid DNA was mixed with 40 µl competent yeast cells, and electroporated at 1.5 kV using a Stratagene electroporator 1000 in a 0.2 mm cuvette. The cells were mixed with 500 µl ice-cold 1 M sorbitol immediately after the pulse and plated on the corresponding solid selective YC-medium containing 1 M sorbitol.

Cloning of pRS316-C1/2_{CUP1}. A sequence containing two polypeptides C1 and C2 was first assembled by PCR using a set of primers as described by Stemmer *et al* (cf. Oakley M.G., and Kim P.S. (1998), Biochemistry 37, 12603-12610; Oakley M.G, and Hollenbeck J.J. (2001), Curr Opin Struct Biol 11, 450-457; Stemmer W.P., Crameri A., Ha K.D., Brennan T.M., Heynecker H.L. (1995), Gene 164, 49-53). In short, the primers were mixed in an equimolar concentration (12.5 µM of each primer) and assembled in 55 cycles of denaturation (94°C, 30 s), primer annealing (52°C, 30 s) and extension (72°C, 30 s) using 0.1 unit/ µl Pwo polymerase and 0.5 mM of each dNTP in the gene assembly buffer (10 mM Tris-HCl, pH 8.8, 2.2 mM MgCl₂, 50 mM KCl and 0.1 % Triton X-100). The double gene was then amplified out of this reaction using Pwo polymerase with the 5'- primer PTP116 that contains an *Eco*RI site and the 3'-primer PTP111 that contains a *Sal*I site. The PCR product was cleaved with *Eco*RI and *Sal*I and cloned into pRS316, resulting in pRS316-C1/2 (cf. Sikorski R.S, and Hieter, P. (1989), Genetics 122, 19-27). The final construct contained the sequences for an N-terminal FLAG tag, the polypeptide C1 followed by a five-residue linker, an *Hpa*I blunt end restriction site and a six-residue-linker followed by the polypeptide C2 with a C-terminal HA tag. C1 and C2 are two peptides that associate into an antiparallel-coiled coil (cf. Oakley M.G., and Kim P.S. (1998), Biochemistry 37, 12603-12610; Oakley M.G, and Hollenbeck J.J. (2001), Curr Opin Struct Biol 11, 450-457). The sequence of the P*_{CUP1}*-promoter was then cleaved out of the plasmid pAGTM2-Dha with *Bam*HI and *Eco*RI and positioned upstream of the C1/ C2 cassette in pRS316-C1/2, resulting in pRS316-C1/2*_{CUP1}*.

Cloning of pRS315_{CUP1} and of pRS316_{CUP1}. The pRS315-derived vector was constructed for an easy cloning of the different Nₜᵣₚ-*SEC62* constructs, whereas the pRS316-derived vector was constructed for an easy cloning of the different *SEC63*-Cₜᵣₚ constructs (cf. Sikorski R.S, and Hieter, P. (1989), Genetics 122, 19-27). The sequence of the P*_{CUP1}*-promoter of the plasmid pAGTM2-Dha was amplified by PCR with the primers PTP181 and PTP182. The gene of *ECFP* was amplified by PCR out of pLP-ECFP-C1 with the primers PTP183 and PTP184. Both fragments were then combined by overlap extension PCR using the 5'-primer PTP181 that contains a *Bam*HI site and the 3'-primer PTP184 that contains a *Sal*I site, so that the P*_{CUP1}*-promoter is upstream of ECFP (cf. Ho S.N. et al. (1989), Gene 1989, 51-59). The partially homologous primers PTP182 and PTP183 contain the sequence of the restriction sites *EcoR*I, *Bgl*II and *Avr*II to allow a versatile cloning of genes downstream of the P*_{CUP1}*-promoter. The final fragment consisting of P*_{CUP1}-*promoter and *ECFP* was then cloned into pRS315 or pRS316 with *Bam*HI and *Sal*I, resulting in pRS315*_{CUP1}* or pRS316*_{CUP1}* (cf. Sikorski R.S, and Hieter, P. (1989), Genetics 122, 19-27).

To generate split-protein sensors based on Trp1p (split-Trp) we adapted an approach originally developed by Graf and Schachmann for creating random circular permutations of proteins (cf. Graf, R., and Schachman, H.K. (1996), Proc Natl Acad Sci U S A 93, 11591-11596). Using PCR, the *TRP1* gene of *Saccharomyces cerevisiae* was first rearranged so that it started with residue 63 and its former start codon was fused to the stop codon via a linker sequence encoding a unique *Avr*II restriction site. The N- and the C- terminal domains of TRP1 were therefore amplified separately out of the plasmid pY-ESTrp2 (Invitrogen) with the primers PTP113/ 115 and PTP112/114, respectively, and recombined using overlap extension PCR with the primers PTP112 and PTP115 (cf. Ho S.N. et al. (1989), Gene 1989, 51-59). This rearrangement was performed to avoid unwanted isolation of wild-type gene in the subsequent selections. At the same time, a HindIII restriction site was introduced via the PCR primers at the newly generated N- and C- termini by introducing a silent mutation in the gene at around amino acid 63. Since the direct digestion of PCR products in former experiments yielded a product that did not ligate efficiently, the rearranged gene was first inserted into a high-copy plasmid (pAK400) and, after amplification of the vector DNA, cut out with HindIII. The rearranged gene was then incubated with T4 DNA ligase at 16°C for 14 h at a DNA concentration of 0.14 mg/ ml, leading to the formation of circular DNA as well as dimers and higher oligomers. After inhibition of the ligase at 65°C for 20 min and desalting of the solution using a microcon PCR column, the ligation products were incubated with DNaseI (∼ 1.2 units/ mg DNA) in 50 mM Tris-HCl, pH 7.5, 1 mM MnCl₂ at 25°C for six minutes. The exact conditions for the DNaseI reactions were determined immediately before the digestion in small test reactions. The DNaseI reaction was stopped by phenol extraction and ethanol precipitation. After incubation of the digested DNA with T4 DNA ligase and T4 polymerase to repair nicks, gaps and to flush the ends of the fragments, DNA fragments corresponding to the size of the original gene were isolated by gel electrophoresis. These fragments were ligated into the pRS316-based yeast expression vector pRS316-Cl/2*_{CUP1}* that was cleaved with HpaI and dephosphorylated according to standard protocols. In the resulting vector, the C-terminal half of *TRP1* is fused to a gene encoding for a FLAG tag, a polypeptide C1 and a five-residue linker sequence and is expressed under the control of the P*_{CUP1}*-promoter. The N-terminal half of *TRP1* is fused to a gene encoding for a six-residue linker sequence, the polypeptide C2 and a HA tag. The sequences of the peptides C1 and C2, including epitope tag and linker are:
C1: MDYKDESGQALEKELAQNEWELQALEKELAQLEKELQAGSGSG,
   (SEQ ID NO: 19)
C2: GGSGSGQALKKKLAQLKWKLQALKKKNAQLKKKLQAGSYPYDVPDYAAFL,
   (SEQ ID NO: 20)

After transformation in XL1Blue, resulting in a library with about 3 × 10⁴ independent clones, the bacteria were scratched from the plate, and the plasmids isolated and linearized with *Avr*II. To insert a terminator for the C-terminal fragment and a promoter for the N-terminal fragment, a DNA fragment was constructed by PCR consisting of the *CYC1* terminator, a geneticin resistance gene, the P*_{GAL1}*-promoter and flanking regions of about 50 base pairs at the 5'-and 3'-ends homologous to the original N and C termini of Trp1p. The CYC1-terminator was amplified out of pYESTrp2 with the primers PTP107 and PTP120, whereas the cassette containing the geneticin resistance gene and the P*_{GAL1}-*promoter was amplified out of pFA6a-GAL1 with the primers PTP108 and PTP121. Both fragments were combined by overlap extension PCR using the primers PTP120 and PTP121 (cf. Ho S.N. et al. (1989), Gene 1989, 51-59). The linearized vector (0.3 µg) and the PCR fragment (3 µg) were then co-transformed in chemically competent EGY48 cells and plated on plates lacking uracil but containing geneticin (500 µg/ml) to select for insertion of the PCR fragment into the linearized vector through homologous recombination (cf. Oldenburg et al. (1997), Nucleic Acids Res 25, 451-452). Chemically competent yeast cells were prepared as described by standard protocols. The homologous recombination also suppressed the predominant isolation of *TRP1* genes that were cut near the original N or C terminus. In the final construct, the C-terminal fragment fused to C1 (C1-Cₜᵣₚ) is under the control of the inducible but leaky P*_{CUP1}*-promoter and the N-terminal fragment fused to C2 (Nₜᵣₚ-C2) is under the stringent control of the P*_{GAL1}*-promoter. After 3 days of incubation at 30°C, approximately 1600 colonies were isolated and subsequently replica-plated on plates lacking uracil and tryptophan but containing geneticin (250 µg/ ml), galactose (2 %) and CuSO₄ (0.1 mM). After replica plating, 45 colonies were able to complement tryptophan auxotrophy. Approximately half of those 45 colonies required the presence of galactose and CuSO₄ to grow on plates lacking tryptophan and twelve of these clones were then analyzed by DNA sequencing (Figure 2). Five of the twelve analyzed clones lead to the expression of Trp1p fragments in which both fragments were fused in frame to the polypeptides C1 and C2 (marked with an asterisk in Figure 2). Seven of the twelve clones were out of frame with C1 or C2. These frame shifts resulted in the replacement of C2 in split-Trp¹³⁵ and split-Trp¹⁷⁰ with a peptide of 66 residues possessing the sequence
DLDQVRHLRRSWRSLSGNCKLLRRRMPSLRRSSRLEVTHMFQITLHFYKSTSRGGPVPSFCSL and in split-Trp¹⁸⁰, split-Trp¹⁹⁸, split-Trp²⁰³ and split-Trp^{204b} with a peptide of 10 residues possessing the sequence (E/Q)RWIWIRSGT. It is assumed that Nₜᵣₚ and Cₜᵣₚ of these clones associate spontaneously without the help of interacting proteins. In split-Trp⁴⁴ and split-Trp^{204b} the mutation Gly8Cys was introduced during the fragmentation procedure. However, the influence of this mutation seems to be of minor importance as the deletion of the first ten amino acids still allowed split-Trp⁷⁷ to complement tryptophan auxotrophy (Figures 2 and 3).

For split-Trp⁴⁴, split-Trp⁵³, split-Trp¹⁸⁷, split-Trp^{204b} and split-Trp⁷⁷ the sequence encoding Nₜᵣₚ-C2 was deleted from the plasmid using *Bgl*II and *Sal*I and replaced with a PCR fragment encoding only the corresponding Nₜᵣₚ fragment. The resulting constructs were then retransformed into EGY48 (Figure 3). To test whether the *trp1* complementation depends on the presence of both Trp1p fragments we repeated the growth assays on plates lacking tryptophan and galactose but containing glucose and copper, thereby repressing the expression of Nₜᵣₚ-C2. Of the five clones tested, only split-Trp⁷⁷ conferred tryptophan auxotrophy to the *trp1* yeast in the presence of glucose by itself, indicating that its large C-terminal fragment spanning residues 11-224 already possesses enzymatic activity. On galactose, split-Trp⁹⁴, split-Trp¹⁸⁷ and split-Trp⁷⁷ complemented tryptophan auxotrophy at 30°C and 23°C, whereas split-Trp⁵³ and split-Trp^{204b} complemented tryptophan auxotrophy only at 23°C (Figure 3).

The deletion of C2 abolished the capacity of the four clones split-Trp⁴⁴, split-Trp⁵³, split-Trp¹⁸⁷, split-Trp^{204b} to complement tryptophan auxotrophy (Figure 3). This finding demonstrates that the formation of a functional Trp1p from these fragments indeed depends on the fusion to a pair of interacting polypeptides.

Since the structure of Trp1p from *S. cerevisiae* has not yet been solved, we aligned its sequence with the sequences of the N-(5'-phosphoribosyl)-anthranilate isomerases from *E. coli* (ePRAI) and *Thermotoga maritima* (tPRAI), and identified the fragmentation sites in the known crystal structures of the homologous enzymes (Figure 4). The fragmentation site of split-Trp⁴⁴ lies in one of the active site loops between β2 and α2, two residues away from an arginine residue that interacts with the carboxyl group of the substrate N-(5'-phosphoribosyl)-anthranilate. Although combinatorial mutagenesis experiments have indicated that turn sequences in general are highly mutable in (β/α)₈-barrels, the vicinity of this position to an active site residue would not have made it an obvious candidate for a fragmentation site (cf. Silverman, J.A., Balakrishnan, R., and Harbury, P.B. (2001), Proc Natl Acad Sci U S A 98, 3092-3097). In split-Trp¹⁸⁷ and split-Trp⁵³ the fragmentation sites are located in α-helices α7 and α2 of the (β/α)₈-barrel, respectively. This appears plausible in hindsight with the mutability of α-helical residues in combinatorial mutagenesis experiments on (β/α)₈-barrels and with earlier random circular permutation experiments of other folds in which new termini were introduced into α-helices (cf. Silverman, J.A., Balakrishnan, R., and Harbury, P.B. (2001), Proc Natl Acad Sci U S A 98, 3092-3097; Graf, R., and Schachman, H.K. (1996), Proc Natl Acad Sci U S A 93, 11591-11596). Furthermore, α-helix α2 is extended by nine amino acids in Trp1p compared to ePRAI and tPRAI, making it plausible that the introduction of a fragmentation site could be tolerated without significantly affecting the activity or the folding of the (β/α)₈-barrel. Particularly interesting is split-Trp^{204b}, in which a stretch of eight amino acids (205-212), including four highly conserved residues, is deleted from Trp1p. This results in a very short Cₜᵣₚ of only twelve residues that is fused to C1, corresponding to α-helix α8 in the structure of tPRAI and ePRAI. The eight deleted amino acids form a loop in the vicinity of the active site, directly after the short α-helix α8'. Helix α8' is believed to participate in the binding of the phosphate group of the substrate and is not present in the regular structures of other (β/α)₈-barrels (cf. Eder, J., and Kirschner, K. (1992), Biochemistry 31, 3617-3625; Hennig, M., Sterner, R., Kirschner, K., and Jansonius, J.N. (1997), Biochemistry 36, 6009-6016). While split-Trp^{204b} complements tryptophan auxotrophy only at 23°C, indicating a decreased stability of the split enzyme, this finding nevertheless questions the significance of this loop with its four completely conserved residues in the function of N-(5'-phopsphoribosyl)-anthranilate isomerases. However, it is unknown how much residual Trp1p activity is sufficient to complement tryptophan auxotrophy in yeast and a more detailed interpretation of this finding will therefore require the kinetic characterization of Split-Trp^{204b} in in vitro assays. Eder and Kirschner have shown that the N-terminal fragment 1-167 folds in the absence of its C-terminal partner (cf. Eder, J., and Kirschner, K. (1992), Biochemistry 31, 3617-3625). Furthermore, it has been proposed that this N-terminal subdomain is an intermediate in the folding of Trp1p (cf. Silverman, J.A., Balakrishnan, R., and Harbury, P.B. (2001), Proc Natl Acad Sci U S A 98, 3092-3097; Kirschner, K., Szadkowski, H., Henschen, A., and Lottspeich, F. (1980), J Mol Biol 143, 395-409; Jasanoff, A., Davis, B., and Fersht, A.R. (1994), Biochemistry 33, 6350-6355; Silverman, J.A., and Harbury, P.B. (2002), J Mol Biol 324, 1031-1040; Sanchez del Pino, M.M., and Fersht, A.R. (1997), Biochemistry 36, 5560-5565). In agreement with these studies all of the selected split-Trp pairs that spontaneously assemble into a functional protein possess relatively large N-terminal fragments, incorporating at least the first five (β/α)-motives. This observation suggests that a spontaneous assembly of Trp1p fragments depends on the presence of a folded N-terminal domain and that the location of the fragmentation site reflects the folding pathway of the natural protein. Shorter N-terminal fragments such as ⁴⁴Nₜᵣₚ and ⁵³Nₜᵣₚ might not fold independently and the chances to spontaneously reconstitute active protein from unfolded fragments without induced proximity would be greatly diminished. Noteworthy, most of the isolated split-Trp pairs that reassemble spontaneously consist of Trp1p fragments that overlap for at least 13 residues. This overlap prevents us to exactly localize the fragmentation site from the sequence data (Figure 2). An exception is split-Trp¹³⁵ where, according to the structure of tPRAI, the fragmentation site is located in a loop at the N-terminal side of the (β/α)₈-barrel.

### Detection of membrane protein interactions using split-Trp sensors

An important application for new split-protein sensors will lie in the detection and characterization of protein-protein interactions occurring at the membranes of intracellular organelles and the cell membranes. To test whether the split-Trp system operates at the membrane, the interaction-dependent split-Trp pairs were attached to the membrane proteins Sec62p and Sec63p (Figure 4) (cf. Panzner, S., Dreier, L., Hartmann, E., Kostka, S., and Rapoport, T.A. (1995), Cell 81, 561-570; Deshaies, R.J., and Schekman, R. (1989), J Cell Biol 109, 2653-2664; Wittke, S., Dunnwald, M., and Johnsson, N. (2000), Mol Biol Cell 11, 3859-3871). Sec62p and Sec63p directly bind to each other and are part of the heptameric Sec-complex that is responsible for translocating proteins posttranslationally across the membrane of the endoplasmic reticulum (ER) (Figure 5A). Briefly, *SEC62* was fused to the 3'-end of the N-terminal fragment of the four split-Trp systems, allowing for the expression of ⁴⁴Nₜᵣₚ-Sec62p, ⁵³Nₜᵣₚ-Sec62p, ¹⁸⁷Nₜᵣₚ-Sec62p and ^{204b}Nₜᵣₚ-Sec62p. *SEC63* was fused to the 5'-end of the corresponding C-terminal fragments, allowing for the expression of Sec63p-⁴⁴Cₜᵣₚ, Sec63p-⁵³Cₜᵣₚ, Sec63p-¹⁸⁷Cₜᵣₚ and Sec63p-^{209b}Cₜᵣₚ.

To monitor the interaction between Sec62p and Sec63p, *trp1* yeast strains expressing pairs of matching Nₜᵣₚ-Sec62p and Sec63p-Cₜᵣₚ fusion proteins were spotted on selective plates lacking tryptophan (Figure 5). Strains co-expressing ⁴⁴Nₜᵣₚ-Sec62p/ Sec63p-⁴⁴Cₜᵣₚ, ¹⁸⁷Nₜᵣₚ-Sec62p/ Sec63p-¹⁸⁷Cₜᵣₚ and ^{204b}Nₜᵣₚ-Sec62p/ Sec63p-^{204b}Cₜᵣₚ were able to grow on plates lacking tryptophan at 23°C but not at 30°C. Only small colonies were detected after 7 days for ¹⁸⁷Nₜᵣₚ-Sec62p/ Sec63p-¹⁸⁷Cₜᵣₚ and after 10 days for ^{204b}Nₜᵣₚ-Sec62p/ Sec63p-^{204b}Cₜᵣₚ, whereas strains co-expressing ⁴⁴Nₜᵣₚ-Sec62p/ Sec63p-⁴⁴Cₜᵣₚ grew significantly faster. No growth at all was observed for strains expressing ⁵³Nₜᵣₚ-Sec62p/ Sec63p-⁵³Cₜᵣₚ. To verify that the observed complementation of tryptophan auxotrophy is a result of the interaction between the Sec62p and Sec63p moieties of the fusion proteins, we fused the C-terminal fragments of split-Trp⁴⁴ and split-Trp¹⁸⁷ to the cytoplasmic site of Ste14p (Figure 4B). Ste14p is a membrane protein of the ER that is known to interact with neither Sec62p nor Sec63p (Figure 4B) (cf. Wittke, S., Lewke, N., Muller, S., and Johnsson, N. (1999), Mol Biol Cell 10, 2519-2530). No growth on plates lacking tryptophan was observed when matching pairs of Sec62p and Ste14p fusion proteins were co-expressed at 23°C or 30°C for 10 days (Figure 5). The cellular amount of Ste14p-⁴⁴Cₜᵣₚ is roughly 2-3 fold lower than the amount of Sec63p-⁴⁴Cₜᵣₚ as determined by western blotting (data not shown). Since this relatively small effect cannot account for the clear growth difference between the strains expressing either ⁴⁴Nₜᵣₚ-Sec62p/ Sec63p-⁴⁴Cₜᵣₚ or ⁴⁴Nₜᵣₚ-Sec62p/Ste14p-⁴⁴Cₜᵣₚ, we conclude that the ⁴⁴Nₜᵣₚ-Sec62p/ Sec63p-⁴⁴Cₜᵣₚ interaction signal is specific.

In more detail, the gene of *SEC62* was amplified by PCR from yeast EGY48 genomic DNA and combined by overlap extension PCR with the N-terminal fragments of split-Trp⁴⁴, split-Trp⁵³, split-Trp¹⁸⁷ and split-Trp^{204b}, yielding ⁴⁴Nₜᵣₚ-*SEC62*, ⁵³Nₜᵣₚ-*SEC62*, ¹⁸⁷Nₜᵣₚ-*SEC62* and ^{214b}Nₜᵣₚ-*SEC62*. At the same time, a 6 × His tag was introduced at the 5'-end of Nₜᵣₚ. The Nₜᵣₚ genes and *SEC62* are connected by a sequence coding for a six-residue linker (GGSGSG). The four Nₜᵣₚ-*SEC62* PCR products were isolated by gel electrophoresis and ligated in a pRS315-derived expression vector (LEU2) (pRS315*_{CUP1}*) under the control of the P*_{CUP1}*-Promoter. Towards this aim, the vector was cleaved with *Bgl*II and *Sal*I and the *ECFP* gene was replaced by the corresponding Nₜᵣₚ-*SEC62* construct.

The genes of *SEC63* and *STE14* were amplified by PCR from yeast EGY48 genomic DNA and combined by overlap extension PCR with the C-terminal fragments of split-Trp⁴⁴, split-Trp⁵³, split-Trp¹⁸⁷ and split-Trp^{204b}. At the same time, a 6 × His tag was introduced at the 3'-end of Cₜᵣₚ, yielding *SEC63*-⁴⁴Cₜᵣₚ-His, *SEC63*-⁵³Cₜᵣₚ-His, *SEC63*-¹⁸⁷Cₜᵣₚ-His, *SEC63*-^{204b}Cₜᵣₚ-His, *STE14*-⁴⁴Cₜᵣₚ-His and *STE14-*¹⁸⁷Cₜᵣₚ-His. *SEC63* and the Cₜᵣₚ-His genes are connected by a sequence coding for a six-residue linker (GGSGSG). The different *SEC63*-Cₜᵣₚ-His and *STE14*-Cₜᵣₚ-His PCR products were isolated by gel electrophoresis and ligated into a pRS316-derived vector (*URA3*) (pRS316*_{CUP1}*, *vide supra*) under the control of the P*_{CUP1}-*promoter. To replace the 6 × His tag by the more sensitive HA tag the genes of the different *SEC63*-Cₜᵣₚ-His and *STE14*-Cₜᵣₚ-His constructs were amplified by PCR with a 3'-primer that contains an HA tag and cloned into pRS316*_{CUP1}*. All *SEC63* and *STE14* fusions contained an HA tag fused to the C terminus of Trp1p. The vector was cleaved with *Bgl*II and *Sal*I and the *ECFP* gene was replaced with the corresponding *SEC63*-Cₜᵣₚ and *STE14*-Cₜᵣₚ constructs. All constructs were verified by DNA sequencing.

Expression of Nₜᵣₚ-Sec62p fusion proteins. Expression and functionality of the Nₜᵣₚ-Sec62p fusion proteins was confirmed by complementation of the temperature-sensitive yeast strain RSY529 (MATα his4 leu2-3,112 ura3-52 sec62-1) (cf. Rothblatt J.A. et al. (1989), J Cell Biol 109, 2641-2652). RSY529 contains an endogenous temperature-sensitive variant of Sec62p. A colony of RSY529 cells transformed with either pRS315 or a pRS315-derived vector expressing ⁴⁴Nₜᵣₚ-Sec62p, ⁵³Nₜᵣₚ-Sec62p, ¹⁸⁷Nₜᵣₚ-Sec62p or ^{204b}Nₜᵣₚ-Sec62p was resuspended in 1 ml water and 5 µl were spotted on YC-L medium containing 0.1 mM CuSO₄ to induce the expression of the fusion proteins and incubated at 30°C and 38°C for 6 d to control for the complementation of the temperature sensitivity of RSY529.

Expression of Sec63p-Cₜᵣₚ and Ste14p-Cₜᵣₚ fusion proteins. The expression of the different Sec63p-Cₜᵣₚ and Ste14p-Cₜᵣₚ fusion proteins was verified by immunoblotting using antibodies against the HA tag at the C terminus of Trp1p. Towards this aim, an overnight culture of yeast EGY48 cells containing one of the Sec63p-Cₜᵣₚ or Ste14p-Cₜᵣₚ fusion proteins was diluted in 10 ml selective medium YC-U to an OD₆₀₀ ∼ 0.8 and grown for 3 h at 30°C and 220 rpm. Protein expression was induced by adding CuSO₄ to a final concentration of 0.1 mM. After 3 h of expression at 30°C and 220 rpm, the cell solution (same volume at same OD when different samples were compared) was centrifuged at 4300 rpm for 10 minutes and the pellet resuspended in 150 µl yeast lysis buffer (50 mM HEPES, pH 7.5, 150 mM NaCl, 5 mM EDTA, 1 % Triton X-100) containing 1 % (v/ v) protease inhibitor cocktail and 0.5 mM PMSF. 200 µl glass beads were added and the solution was vortexed at full speed for 3 × 30 s and cooled on ice in between the vortexing steps. The glass beads and the cell debris were pelleted by centrifugation for 30 s at 13000 rpm and the supernatant was mixed with an appropriate volume of 5 × SDS sample buffer (50 % glycerol, 7.5 % SDS, 250 mM Tris-HCl, pH 8.0, 0.5 % Bromphenol blue, 12.5 mM 2-Mercaptoethanol). Proteins were denatured for 3 min at 95°C. Aliquots were analysed by Western blotting (12 % SDS-PAGE) as described by standard protocols. After blotting, the nitrocellulose membrane was incubated with 3 % dry milk in TBST (10 mM Tris-HCl, 150 mM NaCl, pH 7.9, 0.05 % Tween 20) to block unspecific antibody binding. Expression of Sec63p-Cₜᵣₚ or Ste14p-Cₜᵣₚ fusion constructs was controlled by incubation of the membrane with the primary anti-HA antibody 1:7500 in TBST (10 mM Tris-HCl, 150 mM NaCl, pH 7.9, 0.05 % Tween 20). An anti mouse-HRP antibody conjugate was used 1:7500 in TBST (10 mM Tris-HCl, 150 mM NaCl, pH 7.9, 0.05 % Tween 20) as secondary antibody. Detection was done on a Kodak Image Station 440CF using the NEN Renaissance kit, a luminol-based chemiluminescence system.

The present data demonstrate that in particular split-Trp⁹⁴ is well suited for the detection of protein-protein interactions between membrane proteins. Interestingly, yeast cells co-expressing ⁹⁴Nₜᵣₚ-Sec62p and Sec63p-⁴⁴Cₜᵣₚ require lower growth temperatures for the complementation of tryptophan auxotrophy than the cells expressing the corresponding C1 and C2 coiled coil fusions. This effect might be due to a more favorable orientation of the N- and C-terminal Trp1p fragments in the antiparallel-coiled coil than in the Sec62p/ Sec63p complex.

In conclusion, we have used directed evolution to convert N-(5'-phosphoribosyl)-anthranilate isomerase into a split-protein sensor. In coupling the interaction of cytosolic and membrane proteins to a simple growth assay, the split-Trp system possesses all the necessary features to complement already existing systems to measure and screen for new protein interactions. This split-Trp approach may be used in identifying partners of medically relevant targets, e.g. in three-hybrid assays and protein/small molecule interaction assays. Furthermore, the evolutionary approach introduced here is generally applicable to other enzymes. By generating novel split-protein sensors that are based on proteins functioning in the matrix of e.g. the mitochondrium, the peroxisome or the lumen of the secretory path, this evolutionary approach will help to overcome the lack of techniques to measure protein interactions in the interior of these organelles. Finally, the analysis of the different split-Trp pairs that either spontaneously assemble into a functional (β/α)₈-barrel or need to be fused to interacting proteins to yield folded protein supports the hypothesis that a large N-terminal subdomain of Trp1p is an important intermediate in the folding of the (β/α)₈- barrel.

### Further experimental details

For the various PCR- and gene assembly reactions, if not already noted explicitly above, the following primers and templates were used.

Primers used for Nₜᵣₚ-constructs (cf. attached sequence listing for details):

| **construct** | **5'-primer** | **3'-primer** | **template(s)** | **cloning** s**ites** |
|---|---|---|---|---|
| ⁴⁴Nₜᵣₚ | PTP193 SEQ ID NO: 55 | PTP146 SEQ ID NO: 65 | split-Trp⁴⁴ | - |
| ⁴⁴Nₜᵣₚ-HA | PTP199 SEQ ID NO: 59 | PTP146 SEQ ID NO: 65 | split-Trp⁴⁴ | - |
| ⁵³Nₜᵣₚ | PTP193 SEQ ID NO: 55 | PTP170 SEQ ID NO: 43 | split-Trp⁵³ | - |
| ¹⁸⁷Nₜᵣₚ | PTP193 SEQ ID NO: 55 | PTP172 SEQ ID NO: 45 | split-Trp¹⁸⁷ | - |
| ²⁰⁴Nₜᵣₚ | PTP193 SEQ ID NO: 55 | PTP174 SEQ ID NO: 47 | split-Trp^{204b} | - |
| SEC62 | PTP147 SEQ ID NO: 66 | PTP188 SEQ ID NO: 53 | EGY48 yeast genomic DNA | |
| ⁴⁴Nₜᵣₚ-SEC62 | PTP193 SEQ ID NO: 55 | PTP188 SEQ ID NO: 53 | ⁴⁴Nₜᵣₚ/ SEC62, overlap extension PCR | *Bgl*II/ *Sal*I |
| ⁵³Nₜᵣₚ-SEC62 | PTP193 SEQ ID NO: 55 | PTP188 SEQ ID NO: 53 | ⁵³Nₜᵣₚ/ SEC62, overlap extension PCR | *Bgl*II/ *Sal*I |
| ¹⁸⁷Nₜᵣₚ-SEC62 | PTP193 SEQ ID NO: 55 | PTP188 SEQ ID NO: 53 | ¹⁸⁷Nₜᵣₚ/ SEC62, overlap extension PCR | *Bgl*II/ *Sal*I |
| ^{204b}Nₜᵣₚ-SEC62 | PTP193 SEQ ID NO: 55 | PTP188 SEQ ID NO: 53 | ²⁰⁴Nₜᵣₚ/ SEC62, o-verlap extension PCR | *Bgl*II/ *Sal*I |

Primers used for Cₜᵣₚ-constructs (cf. attached sequence listing for details):

| **construct** | **5'-primer** | **3'-primer** | **template(s)** | **cloning sites** |
|---|---|---|---|---|
| ⁴⁹Cₜᵣₚ-His | PTP155 SEQ ID NO: 41 | PTP194 SEQ ID NO: 56 | split-Trp⁴⁴ | - |
| ⁴⁴Cₜᵣₚ-HA | PTP155 SEQ ID NO: 41 | PTP198 SEQ ID NO: 58 | split-Trp⁴⁴ | - |
| ⁵³Cₜᵣₚ-His | PTP171 SEQ ID NO: 44 | PTP194 SEQ ID NO: 56 | split-Trp⁵³ split-Trp⁵³ | - |
| ¹⁸⁷Cₜᵣₚ-His | PTP173 SEQ ID NO: 46 | PTP194 SEQ ID NO: 56 | split-Trp¹⁸⁷ split-Trp¹⁸⁷ | - |
| ^{204b}Cₜᵣₚ-His | PTP175 SEQ ID NO: 48 | PTP194 SEQ ID NO: 56 | assembly PCR with primers PTP175, PTP176, PTP179, PTP180, PTP191, PTP192 | - |
| SEC63 | PTP189 SEQ ID NO: 54 | PTP154 SEQ ID NO: 40 | EGY48 yeast genomic DNA | - |
| STE14 | PTP195 SEQ ID NO: 57 | PTP157 SEQ ID NO: 42 | EGY48 yeast genomic DNA | - |
| SEC63-⁴⁴Cₜᵣₚ-His | PTP189 SEQ ID NO: 54 | PTP194 SEQ ID NO: 56 | SEC63/ ⁴⁴Cₜᵣₚ-His, overlap extension PCR | *Bgl*II/ *Sal*I |
| SEC63-⁵³Cₜᵣₚ-His | PTP189 SEQ ID NO: 54 | PTP194 SEQ ID NO: 56 | SEC63/ ⁵³Cₜᵣₚ-His, overlap extension PCR | *Bgl*II/ *Sal*I |
| SEC63-¹⁸⁷Cₜᵣₚ-His | PTP189 SEQ ID NO: 54 | PTP194 SEQ ID NO: 56 | SEC63/ ¹⁸⁷Cₜᵣₚ-His, overlap extension PCR | *Bgl*II/ *Sal*I |
| SEC63-^{204b}Cₜᵣₚ-His | PTP189 SEQ ID NO: 54 | PTP194 SEQ ID NO: 56 | SEC63 / ^{204b}Cₜᵣₚ-His, 56 overlap extension PCR | *Bgl*II/ *Sal*I |
| STE14-⁴⁴Cₜᵣₚ-His | PTP189 SEQ ID NO: 54 | PTP194 SEQ ID NO: 56 | STE14/ ⁴⁴Cₜᵣₚ-His, overlap extension PCR | *Bgl*II/ *Sal*I |
| STE14-¹⁸⁷Cₜᵣₚ-His | PTP195 SEQ ID NO: 57 | PTP194 SEQ ID NO: 56 | STE14/ ¹⁸⁷Cₜᵣₚ-His, overlap extension PCR | *Bgl*II/ *Sal*I |
| SEC63-⁴⁴Cₜᵣₚ | PTP195 SEQ ID NO: 54 | PTP194 SEQ ID NO: 58 | SEC63-⁴⁴Cₜᵣₚ-His | *Bgl*II/ *Sal*I |
| SEC63-⁵³Cₜᵣₚ | PTP189 SEQ ID NO: 54 | PTP198 SEQ ID NO: 58 | SEC63-⁵³Cₜᵣₚ-His | *Bgl*II/ *Sal*I |
| SEC63-¹⁸⁷Cₜᵣₚ | PTP189 SEQ ID NO: 54 | PTP198 SEQ ID NO: 58 | SEC63-¹⁸⁷Cₜᵣₚ-His | *Bgl*II/ *Sal*I |
| SEC63-^{204b}Cₜᵣₚ | PTP189 SEQ ID NO: 54 | PTP198 SEQ ID NO: 58 | SEC63-^{204b}Cₜᵣₚ-His | *Bgl*II/ *Sal*I |
| STE14-⁴⁴Cₜᵣₚ | PTP195 SEQ ID NO: 57 | PTP198 SEQ ID NO: 58 | STE14-⁴⁴Cₜᵣₚ-His | *Bgl*II/ *Sal*I |
| STE14-¹⁸⁷Cₜᵣₚ | PTP195 SEQ ID NO: 57 | PTP198 SEQ ID NO: 58 | STE14-¹⁸⁷Cₜᵣₚ-His | *Bgl*II/ *Sal*I |

Primers used for zipper construction (cf. attached sequence listing for details):
- SEQ ID NO: 22:: PTP22
- SEQ ID NO: 23:: PTP23
- SEQ ID NO: 24:: PTP24
- SEQ ID NO: 25:: PTP28
- SEQ ID NO: 26:: PTP29
- SEQ ID NO: 27:: PTP100
- SEQ ID NO: 28:: PTP110
- SEQ ID NO: 29:: PTP111
- SEQ ID NO: 34:: PTP116
- SEQ ID NO: 35:: PTP117
- SEQ ID NO: 36:: PTP118
- SEQ ID NO: 37:: PTP119

Primers used for the copper promoter (cf. attached sequence listing for details):
- SEQ ID NO: 49:: PTP181
- SEQ ID NO: 50:: PTP182
- SEQ ID NO: 51:: PTP183
- SEQ ID NO: 52:: PTP184

Primers used for circular permutation of Trp1p (cf. attached sequence listing for details):
- SEQ ID NO: 30:: PTP112
- SEQ ID NO: 31:: PTP113
- SEQ ID NO: 32:: PTP114
- SEQ ID NO: 33:: PTP115

Primers used for homologous recombination (cf. attached sequence listing for details):
- SEQ ID NO: 63:: PTP107
- SEQ ID NO: 64:: PTP108
- SEQ ID NO: 38:: PTP120
- SEQ ID NO: 39:: PTP121

## Claims

1. A method for the identification of suitable fragmentation sites in a reporter protein, wherein the reporter protein is detectable when active, the method comprising the steps of:
(a) providing a DNA sequence encoding for said reporter protein;
(b) creating a library based on the DNA sequence as defined in (a),
- wherein each individual of said library comprises a randomly created first subsequence of the DNA sequence as defined in (a), encoding for a first subdomain of said reporter protein, and
- wherein each individual of said library comprises a randomly created complementary second subsequence of the DNA sequence as defined in (a), encoding for a complementary second subdomain of said reporter protein;
(c) screening and/or selection for restoration of detectable activity of said reporter protein, when said first subdomain and said complementary second subdomain are brought into close proximity;
(d) identifying said first subdomain and/or said first subsequence, and said complementary second subdomain and/or said complementary second subsequence, that lead to restoration of detectable activity of said reporter protein.

2. A method according to claim 1, wherein the reporter protein is detectable in vivo and/or in vitro, both as full length protein and when actively reassembled by a first subdomain and a complementary second subdomain, by a means chosen from the group consisting of color assays and growth assays.

3. A method according to one of claims 1 or 2, wherein individuals of the library as defined in (b) are either prokaryotic or eukaryotic host cells, comprising:
- both said first subsequence and said complementary second subsequence in one and the same expression vector, suitable for (co-)expression of said first subsequence and said complementary second subsequence in vivo; or
- said first subsequence in a first expression vector suitable for (co-)expression of said first subsequence, and said complementary second subsequence in a second expression vector suitable for (co-)expression of said complementary second subsequence.

4. A method according to one of claims 1 to 3, wherein screening for restoration of detectable activity of said reporter protein, when said first subdomain and said complementary second subdomain are brought into close proximity as defined in (c), comprises the following steps:
- creating a first fusion subsequence comprising the first subsequence of said reporter protein as defined in (b), fused to an oligonucleotide encoding for a first protein or peptide,
- creating a second fusion subsequence comprising the complementary second subsequence of said reporter protein as defined in (b), fused to an oligonucleotide encoding for a second protein or peptide, wherein said first protein or peptide and said second protein or peptide are known to interact.

5. A method according to one of claims 1 to 4, wherein said first protein or peptide and said second protein or peptide align to each other in an anti-parallel coiled coil orientation.

6. A method according to claim 5, wherein said first protein or peptide and said second protein or peptide are leucine zippers.

7. A method according to one of claims 4 to 6, wherein said first fusion subsequence and said second subsequence are created by blunt end ligation.

8. A method according to claim 7, wherein said first fusion subsequence and said second fusion subsequence each comprise
- a linker sequence in between said first subsequence (or said second subsequence, respectively) and said oligonucleotide encoding for a first protein or peptide (or said oligonucleotide encoding for a second protein or peptide, respectively);
- at least one tag that allows for verification of the transcription of said first fusion subsequence and said second fusion subsequence.

9. A method according to one of claims 4 to 8, wherein an oligonucleotide is inserted by homologous recombination in between said first subsequence and said second subsequence, encoding for:
- a transcription terminating sequence for terminating transcription of said first or said second subsequence;
- a transcription promoting sequence for initiating transcription of said second or said first subsequence, respectively;
- a marker sequence allowing for control of successful homologous recombination.

10. A method according to one of claims 1 to 9, comprising the steps of:
- creating fragmentation sites in TRP1 using gene cleavage with a unique restriction enzyme RE1 and circularization;
- isolating fragments corresponding to the wild-type length;
- sub-cloning using blunt ends preferably into a pRS316 based yeast expression vector under the control of a copper promoter (pCUB1) and transforming into E. coli, preferably XL1blue;
- recombining and amplifying homologues with a unique restriction site RE2, preferably AvrII, introduced between the original N- and C-termini to allow subsequent linerization of the vector;
- locating two leucine zippers in the plasmid at the 3'- and the 5'-ends of the newly generated N- and C-termini, the zippers being positive and negative charged helices to allow heterodimerization, preferably each heterodimer containing a buried asparagine residue in a position to force antiparallel orientation of the zippers.

11. A recombinant DNA sequence for use in securing expression in a prokaryotic or eukaryotic host cell of a polypeptide product having the primary structural conformation of a first subdomain of a reporter protein or a complementary second subdomain of a reporter protein, wherein detectable activity of said reporter protein is restored, when said first subdomain and said complementary second subdomain are brought into close proximity, and wherein said first and said complementary second subdomain are not subdomains of one of the group of proteins consisting of transcriptional activators, ubiquitin, dihydrofolate reductase, β-lactamase, green fluorescent protein, β-galactosidase, inteins, cAMP cyclase, glycinamide ribonucleotide transformylase, aminoglycoside phosphotransferase, hygromycin B phosphotransferase, luciferase;
wherein
said DNA sequence encodes for a subdomain of a (β/α)₈-barrel enzyme;
and
wherein said DNA sequence is selected from the group consisting of:
(a) the DNA sequences SEQ ID NO 3, 5, 11, 13, 15, 17 or their complementary strands;
(b) DNA sequences which, but for the degeneracy of the genetic code, would hybridize to the DNA sequences defined in (a) and which sequences code for a polypeptide having the same amino acid sequence.

12. A recombinant DNA sequence according to claim 11, wherein said DNA sequence is for use in securing expression in a prokaryotic or eukaryotic host cell of a polypeptide fusion product.

13. A first subdomain of a reporter protein and/or a complementary second subdomain ot a reporter protein, wherein detectable activity of said reporter protein is restored, when said first subdomain and said complementary second subdomain are brought into close proximity, and wherein said first and said complementary second subdomain are not subdomains of one of the group of proteins consisting of transcriptional activators, ubiquitin, dihydrofolate reductase, β-lactamase, green fluorescent protein, β-galactosidase, inteins, cAMP cyclase, glycinamide ribonucleotide transformylase, aminoglycoside phosphotransferase, hygromycin B phosphotransferase, luciferase;
wherein the first subdomain of a reporter protein or a complementary second subdomain of a reporter protein is produced by a method of culturing a host transformed with a recombinant DNA molecule selected from the group consisting of the DNA molecules of claims 11 to 12, wherein said molecules further comprises an expression control sequence, said expression control sequence being operatively linked to said molecule.

14. A first subdomain of a reporter protein or a complementary second subdomain of a reporter protein according to claim 13, wherein the site of fragmentation of said reporter protein into a first subdomain and a complementary second subdomain is identified by a method according to one of claims 1 to 10.

15. A fusion protein comprising a first subdomain of a reporter protein or a complementary second subdomain of a reporter protein according to one of claims 13 to 14, and a further peptide or protein connected thereto in a naturally not occurring combination.

16. A prokaryotic or eukaryotic host cell line, transformed with a recombinant DNA sequence according to one of claims 11 to 12.

17. A host cell line according to claim 16, wherein the host cell line allows for homologous recombination of DNA.

18. A host cell line according to claim 17, which host cell line comprises a yeast cell line.

19. A host cell line according to claim 18, which yeast cell line is chosen from the group consisting of *Saccharomyces cerevisiae* and *Schizosaccharomyces pombe.*

20. A kit of parts, comprising a first and a second DNA-based expression vector, wherein
- said first expression vector contains an expression cassette encoding for a polypeptide product having at least a substantial part of the primary structural confirmation of a first subdomain of a reporter protein according to one of claims 13 to 14; and
- said second expression vector contains an expression cassette encoding for a polypeptide product having at least a substantial part of the primary structural confirmation of a complementary second subdomain of a reporter protein according to one of claims 13 to 14; and
wherein detectable activity of said reporter protein is restored, when said first subdomain and said complementary second subdomain are brought into close proximity,
and wherein said first and said complementary second subdomain are not subdomains of one the group of proteins consisting of transcriptional activators, ubiquitin, dihydrofolate reductase, β-lactamase, green fluorescent protein, β-galactosidase, inteins, cAMP cyclase, glycinamide ribonucleotide transformylase, aminoglycoside phosphotransferase, hygromycin B phosphotransferase, luciferase.

21. A kit of parts according to claim 20, further comprising a suitable prokaryotic or eukaryotic host cell line for expression of said first and second expression vector.

22. A method for detecting an interaction between a first test peptide or protein or a fragment thereof, and a second test peptide or protein or a fragment thereof, the method comprising the steps of:
- providing recombinant DNA sequences according to one of claims 11 or 12 for use in securing expression of a first subdomain of a reporter protein and a complementary second subdomain of a reporter protein;
- fusing an oligonucleotide or a gene encoding for a first test peptide or protein to the DNA sequence encoding for said first subdomain of the reporter protein, thereby creating a first DNA fusion sequence encoding for a fusion protein comprising said first subdomain of the reporter protein and said first test peptide or protein;
- fusing an oligonucleotide or a gene encoding for a second test peptide or protein to the DNA sequence encoding for said complementary second subdomain of the reporter protein, thereby creating a second DNA fusion sequence encoding for a fusion protein comprising said complementary second subdomain of the reporter protein and said second test peptide or protein;
- (co-)expressing said fusion protein comprising said first subdomain of the reporter protein and said first test peptide or protein, and said fusion protein comprising said second complementary subdomain of the reporter protein and said second test peptide or protein in a suitable prokaryotic or eukaryotic host cell;
- screening and/or selecting for restoration of detectable activity of said reporter protein.

23. A method according to claim 22, wherein a library of oligonucleotides or DNA encoding for a set of first test peptides or proteins and/or a library of oligonucleotides or DNA encoding for a set of second test peptides or proteins are fused to said first subdomain of said reporter protein and/or said complementary second subdomain of said reporter protein, respectively.

24. A method according to one of claims 22 to 23, wherein said first test peptide or protein or a fragment thereof, and said second test peptide or protein or a fragment thereof, are peptides or proteins naturally occurring in compartments chosen from the group consisting of cellular membranes, the cytosol, the mitochondrium, the peroxisome and the lumen of the secretory path.

25. A method according to one of claims 22 to 24, wherein said interaction between a first test peptide or protein or a fragment thereof and a second test peptide or protein or fragment thereof is mediated by a chemical inducer of dimerization, which binds either covalently or non-covalently to both said test peptides or proteins or fragments thereof.

26. A method for detecting the interruption of an interaction between a first test peptide or protein or a fragment thereof, and a second test peptide or protein or a fragment thereof, the method comprising the steps of:
- providing recombinant DNA sequences according to one of claims 11 or 12 for use in securing expression of a first subdomain of a reporter protein and a complementary second subdomain of a reporter protein;
- fusing an oligonucleotide or a gene encoding for a first test peptide or protein to the DNA sequence encoding for said first subdomain of the reporter protein, thereby creating a first DNA fusion sequence encoding for a fusion protein comprising said first subdomain of the reporter protein and said first test peptide or protein;
- fusing an oligonucleotide or a gene encoding for a second test peptide or protein to the DNA sequence encoding for said complementary second subdomain of the reporter protein, thereby creating a second DNA fusion sequence encoding for a fusion protein comprising said complementary second subdomain of the reporter protein and said second test peptide or protein;
- (co-)expressing said fusion protein comprising said first subdomain of the reporter protein and said first test peptide or protein, and said fusion protein comprising said second complementary subdomain of the reporter protein and said second test peptide or protein in a suitable prokaryotic or eukaryotic host cell;
- screening and/or selecting for interruption of interaction of said first subdomain and said second subdomain under the influence of one or more test agents.

27. Use of random circular permutation of a gene and/or the expressed polypeptide derived thereof for the identification of fragmentation sites in a reporter protein for use in a two-hybrid system.

28. Use of a host cell line capable of homologous recombination of DNA, in a method according to one of claims 1 to 10.

## Patentansprüche

1. Verfahren zur Identifizierung geeigneter Fragmentierungsstellen in einem Reporterprotein, wobei das Reporterprotein, wenn aktiv, nachweisbar ist, wobei das Verfahren die folgenden Schritte umfasst:
(a) Bereitstellen einer für das Reporterprotein codierenden DNA-Sequenz;
(b) Erzeugen einer auf der DNA-Sequenz mit der unter (a) angegebenen Bedeutung basierenden Bibliothek,
- wobei jedes Individuum der Bibliothek eine zufällig erzeugte erste Teilsequenz der DNA-Sequenz mit der unter (a) angegebenen Bedeutung umfasst, die für eine erste Subdomäne des Reporterproteins codiert, und
- wobei jedes Individuum der Bibliothek eine zufällig erzeugte komplementäre zweite Teilsequenz der DNA-Sequenz mit der unter (a) angegebenen Bedeutung umfasst, die für eine komplementäre zweite Subdomäne des Reporterproteins codiert,
(c) Screening und/oder Selektion auf Wiederherstellung nachweisbarer Aktivität des Reporterproteins, wenn die erste Subdomäne und die komplementäre zweite Subdomäne in enge Nachbarschaft zueinander gebracht werden;
(d) Identifizieren der ersten Subdomäne und/oder der ersten Teilsequenz sowie der komplementären zweiten Subdomäne und/oder der komplementären zweiten Teilsequenz, die zur Wiederherstellung nachweisbarer Aktivität des Reporterproteins führen.

2. Verfahren nach Anspruch 1, wobei das Reporterprotein in vivo und/oder in vitro sowohl als Volllängenprotein als auch nach aktivem Wiederzusammenbau von einer ersten Subdomäne und einer komplementären zweiten Subdomäne mit einem aus der aus Farbtests und Wachstumstests bestehenden Gruppe gewählten Mittel nachweisbar ist.

3. Verfahren nach einem der Ansprüche 1 oder 2, wobei es sich bei den Individuen der Bibliothek mit der unter (b) angegebenen Bedeutung um entweder prokaryontische oder eukaryontische Wirtszellen handelt, die
- sowohl die erste Teilsequenz als auch die komplementäre zweite Teilsequenz in ein und demselben Expressionsvektor, der sich für die (Co-)Expression der ersten Teilsequenz und der komplementären zweiten Teilsequenz in vivo eignet, oder
- die erste Teilsequenz in einem für die (Co-)Expression der ersten Teilsequenz geeigneten ersten Expressionsvektor und die komplementäre zweite Teilsequenz in einem für die (Co-)Expression der komplementären zweiten Teilsequenz geeigneten zweiten Expressionsvektor umfassen.

4. Verfahren nach einem der Ansprüche 1 bis 3, wobei das Screening auf Wiederherstellung nachweisbarer Aktivität des Reporterproteins, wenn die erste Subdomäne und die komplementäre zweite Subdomäne in enge Nachbarschaft zueinander gebracht werden, wie unter (c) definiert, die folgenden Schritte umfasst:
- Erzeugen einer ersten Fusionsteilsequenz, die die erste Teilsequenz des Reporterproteins mit der unter (b) angegebenen Bedeutung, fusioniert an ein für ein erstes Protein oder Peptid codierendes Oligonukleotid, umfasst,
- Erzeugen einer zweiten Fusionsteilsequenz, die die komplementäre zweite Teilsequenz des Reporterproteins mit der unter (b) angegebenen Bedeutung, fusioniert an ein für ein zweites Protein oder Peptid codierendes Oligonukleotid, umfasst,
wobei bekannt ist, dass das erste Protein oder Peptid und das zweite Protein oder Peptid miteinander wechselwirken.

5. Verfahren nach einem der Ansprüche 1 bis 4, wobei sich das erste Protein oder Peptid und das zweite Protein oder Peptid aneinander in einer antiparallelen "Coiled Coil"-Orientierung ausrichten.

6. Verfahren nach Anspruch 5, wobei es sich bei dem ersten Protein oder Peptid und bei dem zweiten Protein oder Peptid um Leucin-Zipper handelt.

7. Verfahren nach einem der Ansprüche 4 bis 6, wobei die erste Fusionsteilsequenz und die zweite Teilsequenz durch Stumpfe-Enden-Ligation erzeugt werden.

8. Verfahren nach Anspruch 7, wobei die erste Fusionsteilsequenz und die zweite Fusionsteilsequenz jeweils
- eine Linker-Sequenz zwischen der ersten Teilsequenz (bzw. der zweiten Teilsequenz) und dem für ein erstes Protein oder Peptid codierenden Oligonukleotid (bzw. dem für ein zweites Protein oder Peptid codierenden Oligonukleotid),
- wenigstens ein Tag, das die Verifizierung der Transkription der ersten Fusionsteilsequenz und der zweiten Fusionsteilsequenz gestattet, umfassen.

9. Verfahren nach einem der Ansprüche 4 bis 8, wobei ein Oligonukleotid durch homologe Rekombination zwischen der ersten Teilsequenz und der zweiten Teilsequenz eingefügt wird, das für:
- eine Transkriptionsterminationssequenz zum Beenden der Transkription der ersten oder der zweiten Teilsequenz,
- eine Transkriptionspromotionssequenz zum Starten der Transkription der zweiten bzw. der ersten Teilsequenz,
- eine Markersequenz, die die Erfolgskontrolle der homologen Rekombination gestattet, codiert.

10. Verfahren nach einem der Ansprüche 1 bis 9, das die Schritte umfasst:
- Erzeugen von Fragmentierungsstellen in TRP1 unter Verwendung von Genspaltung mit einem einmaligen Restriktionsenzym RE1 und Zirkularisierung;
- Isolieren von der Wildtyp-Länge entsprechenden Fragmenten;
- Subklonieren unter Verwendung stumpfer Enden, vorzugsweise in einen auf pRS316 basierenden Expressionsvektor unter der Kontrolle eines Kupferpromotors (pCUB1) und Transformieren in E. coli, vorzugsweise XL1blue;
- Rekombinieren und Amplifizieren von Homologen mit einer einmaligen Restriktionsstelle RE2, vorzugsweise AvrII, eingeführt zwischen den ursprünglichen N- und C-Termini, um eine anschließende Linearisierung des Vektors zu gestatten;
- Lokalisieren von zwei Leucin-Zippern im Plasmid an den 3'- und den 5'-Enden der neu erzeugten N- und C-Termini, wobei es sich bei den Zippern um positiv und negativ geladene Helices handelt, um eine Heterodimerisierung zu gestatten, wobei jedes Heterodimer vorzugsweise einen verborgenen Asparaginrest in einer Position enthält, so dass eine antiparallele Orientierung der Zipper erzwungen wird.

11. Rekombinante DNA-Sequenz zur Verwendung bei der Sicherstellung der Expression eines die Primärstrukturkonformation einer ersten Subdomäne eines Reporterproteins oder einer komplementären zweiten Subdomäne eines Reporterproteins aufweisenden Polypeptidprodukts in einer prokaryontischen oder eukaryontischen Wirtszelle, wobei eine nachweisbare Aktivität des Reporterproteins wiederhergestellt wird, wenn die erste Subdomäne und die komplementäre zweite Subdomäne in enge Nachbarschaft zueinander gebracht werden, und wobei es sich bei der ersten und der komplementären zweiten Subdomäne nicht um Subdomänen eines Proteins aus der Gruppe von Proteinen, bestehend aus Transkriptionsaktivatoren, Ubiquitin, Dihydrofolat-Reduktase, β-Lactamase, grün fluoreszierendem Protein, β-Galactosidase, Inteinen, cAMP-Cyclase, Glycinamid-Ribonukleotid-Transformylase, Aminoglycosid-Phosphotransferase, Hygromycin-B-Phosphotransferase, Luciferase, handelt,
wobei die DNA-Sequenz für eine Subdomäne eines (β/α)₈-Barrel-Enzyms codiert,
und wobei die DNA-Sequenz ausgewählt ist aus der Gruppe bestehend aus:
(a) den DNA-Sequenzen SEQ ID NO 3, 5, 11, 13, 15, 17 oder deren komplementären Strängen;
(b) DNA-Sequenzen, die trotz Degeneration des genetischen Codes an die unter (a) angeführten DNA-Sequenzen hybridisieren würden und die für ein Polypeptid mit der gleichen Aminosäuresequenz codieren.

12. Rekombinante DNA-Sequenz nach Anspruch 11, wobei die DNA-Sequenz bei der Sicherstellung der Expression eines Polypeptidfusionsprodukts in einer prokaryontischen oder eukaryontischen Wirtszelle dient.

13. Erste Subdomäne eines Reporterproteins und/oder komplementäre zweite Subdomäne eines Reporterproteins, wobei eine nachweisbare Aktivität des Reporterproteins wiederhergestellt wird, wenn die erste Subdomäne und die komplementäre zweite Subdomäne in enge Nachbarschaft zueinander gebracht werden, und wobei es sich bei der ersten und der komplementären zweiten Subdomäne nicht um Subdomänen eines Proteins aus der Gruppe von Proteinen, bestehend aus Transkriptionsaktivatoren, Ubiquitin, Dihydrofolat-Reduktase, β-Lactamase, grün fluoreszierendem Protein, β-Galactosidase, Inteinen, cAMP-Cyclase, Glycinamid-Ribonukleotid-Transformylase, Aminoglycosid-Phosphotransferase, Hygromycin-B-Phosphotransferase, Luciferase, handelt,
wobei die erste Subdomäne eines Reporterproteins oder eine komplementäre zweite Subdomäne eines Reporterproteins mit einem Verfahren hergestellt wird, bei dem man einen mit einem aus der Gruppe bestehend aus den DNA-Molekülen der Ansprüche 11 bis 12 ausgewählten rekombinanten DNA-Molekül transformierten Wirt kultiviert, wobei die Moleküle ferner eine Expressionskontrollsequenz umfassen, die in operativer Verknüpfung mit dem Molekül steht.

14. Erste Subdomäne eines Reporterproteins oder komplementäre zweite Subdomäne eines Reporterproteins nach Anspruch 13, wobei die Stelle der Fragmentierung des Reporterproteins in eine erste Subdomäne und eine komplementäre zweite Subdomäne mit einem Verfahren nach einem der Ansprüche 1 bis 10 identifiziert wird.

15. Fusionsprotein, umfassend eine erste Subdomäne eines Reporterproteins oder eine komplementäre zweite Subdomäne eines Reporterproteins nach einem der Ansprüche 13 bis 14 sowie ein damit in einer nicht natürlich vorkommenden Kombination verbundenes weiteres Peptid oder Protein.

16. Prokaryontische oder eukaryontische Wirtszelllinie, transformiert mit einer rekombinanten DNA-Sequenz nach einem der Ansprüche 11 bis 12.

17. Wirtszelllinie nach Anspruch 16, wobei die Wirtszelllinie homologe Rekombination von DNA gestattet.

18. Wirtszelllinie nach Anspruch 17, die eine Hefezelllinie umfasst.

19. Wirtszelllinie nach Anspruch 18, die aus der Gruppe bestehend aus *Saccharomyces cerevisiae* und *Schizosaccharomyces pombe* gewählt wird.

20. Kit-of-parts, umfassend einen ersten und einen zweiten Expressionsvektor auf DNA-Basis, wobei
- der erste Expressionsvektor eine für ein Polypeptidprodukt mit wenigstens einem wesentlichen Teil der Primärstrukturkonformation einer ersten Subdomäne eines Reporterproteins nach einem der Ansprüche 13 oder 14 codierende Expressionskassette enthält und
- der zweite Expressionsvektor eine für ein Polypeptidprodukt mit wenigstens einem wesentlichen Teil der Primärstrukturkonformation einer komplementären zweiten Subdomäne eines Reporterproteins nach einem der Ansprüche 13 oder 14 codierende Expressionskassette enthält und
wobei eine nachweisbare Aktivität des Reporterproteins wiederhergestellt wird, wenn die erste Subdomäne und die komplementäre zweite Subdomäne in enge Nachbarschaft zueinander gebracht werden, und wobei es sich bei der ersten und der komplementären zweiten Subdomäne nicht um Subdomänen eines Proteins aus der Gruppe von Proteinen, bestehend aus Transkriptionsaktivatoren, Ubiquitin, Dihydrofolat-Reduktase, β-Lactamase, grün fluoreszierendem Protein, β-Galactosidase, Inteinen, cAMP-Cyclase, Glycinamid-Ribonukleotid-Transformylase, Aminoglycosid-Phosphotransferase, Hygromycin-B-Phosphotransferase, Luciferase, handelt.

21. Kit-of-parts nach Anspruch 20, ferner umfassend eine geeignete prokaryontische oder eukaryontische Wirtszelllinie zur Expression des ersten und zweiten Expressionsvektors.

22. Verfahren zum Nachweis einer Wechselwirkung zwischen einem ersten Testpeptid oder -protein oder einem Fragment davon und einem zweiten Testpeptid oder -protein oder einem Fragment davon, wobei das Verfahren die folgenden Schritte umfasst:
- Bereitstellen rekombinanter DNA-Sequenzen nach einem der Ansprüche 11 oder 12 zur Verwendung bei der Sicherstellung der Expression einer ersten Subdomäne eines Reporterproteins und einer komplementären zweiten Subdomäne eines Reporterproteins;
- Fusionieren eines Oligonukleotids oder eines Gens, das für ein erstes Testpeptid oder -protein codiert, an die für die erste Subdomäne des Reporterproteins codierende DNA-Sequenz, wodurch eine erste DNA-Fusionssequenz geschaffen wird, die für ein die erste Subdomäne des Reporterproteins und das erste Testpeptid oder -protein umfassendes Fusionsprotein codiert;
- Fusionieren eines Oligonukleotids oder eines Gens, das für ein zweites Testpeptid oder -protein codiert, an die für die komplementäre zweite Subdomäne des Reporterproteins codierende DNA-Sequenz, wodurch eine zweite DNA-Fusionssequenz geschaffen wird, die für ein die komplementäre zweite Subdomäne des Reporterproteins und das zweite Testpeptid oder -protein umfassendes Fusionsprotein codiert;
- (Co-)Exprimieren des die erste Subdomäne des Reporterproteins und das erste Testpeptid oder -protein umfassenden Fusionsproteins und des die komplementäre zweite Subdomäne des Reporterproteins und das zweite Testpeptid oder -protein umfassenden Fusionsproteins in einer geeigneten prokaryontischen oder eukaryontischen Wirtszelle;
- Screening und/oder Selektionieren auf Wiederherstellung nachweisbarer Aktivität des Reporterproteins.

23. Verfahren nach Anspruch 22, wobei eine Bibliothek für einen Satz erster Testpeptide oder -proteine codierender Oligonukleotide oder DNA und/oder eine Bibliothek für einen Satz zweiter Testpeptide oder -proteine codierender Oligonukleotide oder DNA an die erste Subdomäne des Reporterproteins und/oder die komplementäre zweite Subdomäne des Reporterproteins fusioniert wird.

24. Verfahren nach einem der Ansprüche 22 bis 23, wobei es sich bei dem ersten Testpeptid oder -protein oder einem Fragment davon und bei dem zweiten Testpeptid oder -protein oder einem Fragment davon um Peptide oder Proteine handelt, die in Kompartimenten, gewählt aus der Gruppe bestehend aus Zellmembranen, dem Zytosol, dem Mitochondrium, dem Peroxisom und dem Lumen des Sekretionswegs, natürlich vorkommen.

25. Verfahren nach einem der Ansprüche 22 bis 24, wobei die Wechselwirkung zwischen einem ersten Testpeptid oder -protein oder einem Fragment davon und einem zweiten Testpeptid oder -protein oder Fragment davon durch einen chemischen Dimerisierungsinduktor vermittelt wird, der entweder kovalent oder nicht kovalent an beide Testpeptide oder -proteine oder Fragmente davon bindet.

26. Verfahren zum Nachweis der Unterbrechung einer Wechselwirkung zwischen einem ersten Testpeptid oder -protein oder einem Fragment davon und einem zweiten Testpeptid oder -protein oder einem Fragment davon, wobei das Verfahren die folgenden Schritte umfasst:
- Bereitstellen rekombinanter DNA-Sequenzen nach einem der Ansprüche 11 oder 12 zur Verwendung bei der Sicherstellung der Expression einer ersten Subdomäne eines Reporterproteins und einer komplementären zweiten Subdomäne eines Reporterproteins;
- Fusionieren eines Oligonukleotids oder eines Gens, das für ein erstes Testpeptid oder -protein codiert, an die für die erste Subdomäne des Reporterproteins codierende DNA-Sequenz, wodurch eine erste DNA-Fusionssequenz geschaffen wird, die für ein die erste Subdomäne des Reporterproteins und das erste Testpeptid oder -protein umfassendes Fusionsprotein codiert;
- Fusionieren eines Oligonukleotids oder eines Gens, das für ein zweites Testpeptid oder -protein codiert, an die für die komplementäre zweite Subdomäne des Reporterproteins codierende DNA-Sequenz, wodurch eine zweite DNA-Fusionssequenz geschaffen wird, die für ein die komplementäre zweite Subdomäne des Reporterproteins und das zweite Testpeptid oder -protein umfassendes Fusionsprotein codiert;
- (Co-)Exprimieren des die erste Subdomäne des Reporterproteins und das erste Testpeptid oder -protein umfassenden Fusionsproteins und des die komplementäre zweite Subdomäne des Reporterproteins und das zweite Testpeptid oder -protein umfassenden Fusionsproteins in einer geeigneten prokaryontischen oder eukaryontischen Wirtszelle;
- Screening und/oder Selektionieren auf Unterbrechung der Wechselwirkung der ersten Subdomäne und der zweiten Subdomäne unter dem Einfluss eines oder mehrerer Testagentien.

27. Verwendung zufälliger zirkulärer Permutation eines Gens und/oder des davon abgeleiteten exprimierten Polypeptids zur Identifizierung von Fragmentierungsstellen in einem Reporterprotein für die Verwendung in einem Two-Hybrid-System.

28. Verwendung einer zur homologen Rekombination von DNA fähigen Wirtszelllinie in einem Verfahren nach einem der Ansprüche 1 bis 10.

## Revendications

1. Procédé pour identifier des sites de fragmentation appropriés dans une protéine rapporteur, dans lequel la protéine rapporteur est détectable quand elle est active, le procédé comprenant les étapes consistant à :
(a) fournir une séquence d'ADN codant pour ladite protéine rapporteur ;
(b) créer une banque basée sur la séquence d'ADN, telle que définie dans le paragraphe (a),
- dans laquelle chaque individu de ladite banque comprend une première sous séquence, créée de manière aléatoire, de la séquence d'ADN telle que définie dans le paragraphe (a), codant pour un premier sous domaine de ladite protéine rapporteur et
- dans laquelle chaque individu de ladite banque comprend une deuxième sous séquence, créée de manière aléatoire, complémentaire de la séquence d'ADN telle que définie dans le paragraphe (a), codant pour un deuxième sous domaine complémentaire de ladite protéine rapporteur ;
(c) effectuer un criblage et/ou une sélection en vue d'une restitution de l'activité détectable de ladite protéine rapporteur, quand ledit premier sous domaine et ledit deuxième sous domaine complémentaire sont placés en étroite proximité ;
(d) identifier ledit premier sous domaine et/ou ladite première sous séquence ainsi que ledit deuxième sous domaine complémentaire et/ou ladite deuxième sous séquence complémentaire, ce qui conduit à la restitution d'une activité détectable de ladite protéine rapporteur.

2. Procédé selon la revendication 1, dans lequel la protéine rapporteur est détectable *in vivo* et/ou in vitro, dans les deux cas en tant que protéine de longueur totale, et quand elle est réassemblée activement par un premier sous domaine et un deuxième sous domaine complémentaire, via un moyen choisi dans le groupe constitué par des dosages colorimétriques et des dosages de croissance.

3. Procédé selon l'une des revendications 1 ou 2, dans lequel les individus de la banque, telle que définie dans le paragraphe (b), sont des cellules hôtes procaryotes ou eucaryotes comprenant :
- à la fois ladite première sous séquence et ladite deuxième sous séquence complémentaire dans un seul et même vecteur d'expression, étant approprié pour une (co)-expression *in vivo* de ladite première sous séquence et ladite deuxième sous séquence complémentaire ; ou
- ladite première sous séquence dans un premier vecteur d'expression, étant approprié pour une (co)-expression de ladite première sous séquence, et ladite deuxième sous séquence complémentaire dans un deuxième vecteur d'expression, étant approprié pour une (co)-expression de ladite deuxième sous séquence complémentaire.

4. Procédé selon l'une des revendications 1 à 3, dans lequel le criblage en vue de la restitution d'une activité détectable de ladite protéine rapporteur, quand ledit premier sous domaine et ledit deuxième sous domaine complémentaire sont placés en étroite proximité, tel que défini dans le paragraphe (c), comprend les étapes suivantes :
- créer une première sous séquence de fusion comprenant la première sous séquence de ladite protéine rapporteur, telle que définie dans le paragraphe (b), qui est fusionnée avec un oligonucléotide codant pour une première protéine ou un premier peptide,
- créer une deuxième sous séquence de fusion comprenant la deuxième sous séquence complémentaire de ladite protéine rapporteur, telle que définie dans le paragraphe (b), qui est fusionnée avec un oligonucléotide codant pour un(e) deuxième protéine ou peptide,
dans lequel ladite première protéine ou ledit premier peptide ainsi que ladite deuxième protéine ou ledit deuxième peptide sont connus pour entrer en interaction.

5. Procédé selon l'une des revendications 1 à 4, dans lequel ladite première protéine ou ledit premier peptide ainsi que ladite deuxième protéine ou ledit deuxième peptide s'alignent l'un(e) par rapport à l'autre dans une orientation antiparallèle « coiled coil ».

6. Procédé selon la revendication 5, dans lequel ladite première protéine ou ledit premier peptide ainsi que ladite deuxième protéine ou ledit deuxième peptide sont des glissières à leucine.

7. Procédé selon l'une des revendications 4 à 6, dans lequel ladite première sous séquence de fusion et ladite deuxième sous séquence sont créées par ligature d'extrémités franches.

8. Procédé selon la revendication 7, dans lequel ladite première sous séquence de fusion et ladite deuxième sous séquence de fusion comprennent chacune :
- une séquence lieur entre ladite première sous séquence (ou ladite deuxième sous séquence respectivement) et ledit oligonucléotide codant pour une première protéine ou un premier peptide (ou ledit oligonucléotide codant pour un(e) deuxième protéine ou peptide respectivement) ;
- au moins une étiquette qui permet la vérification de la transcription de ladite première sous séquence de fusion et de ladite deuxième sous séquence de fusion.

9. Procédé selon l'une des revendications 4 à 8, dans lequel un oligonucléotide est inséré, par recombinaison homologue, entre ladite première sous séquence et ladite deuxième sous séquence, codant pour :
- une séquence de terminaison de la transcription, destinée à terminer la transcription de ladite première ou ladite deuxième sous séquence ;
- une séquence de favorisation de la transcription, destinée à débuter la transcription de ladite deuxième ou ladite première sous séquence respectivement ;
- une séquence marqueur permettant de contrôler le succès de la recombinaison homologue.

10. Procédé, selon l'une des revendications 1 à 9, comprenant les étapes consistant à :
- créer des sites de fragmentation dans un TRP1, en utilisant une coupure génique avec une enzyme unique de restriction RE1 et une circularisation ;
- isoler des fragments correspondant à la longueur de type sauvage ;
- les sous-cloner en utilisant des extrémités franches, de préférence dans un vecteur d'expression de levure basé sur un pRS316 qui est sous le contrôle d'un promoteur copper (pCUB1), et les transformer en *E*. *coli*, de préférence XL1blue ;
- recombiner et amplifier les homologues avec un site unique de restriction RE2, de préférence AvrII, introduit entre les extrémités N- et C-terminales d'origine pour permettre la linéarisation ultérieure du vecteur ;
- localiser deux glissières à leucine dans le plasmide au niveau des extrémités 3' et 5' des extrémités N- et C- terminales nouvellement générées, les glissières étant des hélices chargées positivement et négativement pour permettre d'obtenir une hétéro-dimérisation, chaque hétérodimère contenant, de préférence, un résidu d'asparagine enterré, dans une position destinée à forcer l'orientation antiparallèle des glissières.

11. Séquence d'ADN recombinant destinée à être utilisée dans la sécurisation de l'expression d'un produit polypeptidique, dans une cellule hôte procaryote ou eucaryote, ayant la conformation structurale primaire d'un premier sous domaine d'une protéine rapporteur ou un deuxième sous domaine complémentaire d'une protéine rapporteur, dans laquelle une activité détectable de ladite protéine rapporteur est restituée lorsque ledit premier sous domaine et ledit deuxième sous domaine complémentaire sont placés en étroite proximité, et dans laquelle ledit premier sous domaine et ledit deuxième sous domaine complémentaire ne sont pas des sous domaines de l'un des groupes de protéines constitué par des activateurs transcriptionnels, l'ubiquitine, une dihydrofolate réductase, une β-lactamase, une protéine fluorescente verte, la β-galactosidase, des intéines, une AMPc cyclase, une glycinamide ribonucléotide transformylase, une aminoglycoside phosphotransférase, une hygromycine B phosphotransférase, la luciférase ;
dans laquelle ladite séquence d'ADN code pour un sous domaine d'une enzyme en tonneau (β/α)₈ ;
et
dans laquelle ladite séquence d'ADN est choisie dans le groupe constitué par :
(a) les séquences d'ADN SEQ ID n° 3, 5, 11, 13, 15, 17 ou leurs brins complémentaires ;
(b) des séquences d'ADN qui s'hybrideraient, sauf pour la dégénérescence du code génétique, avec les séquences d'ADN définies dans le paragraphe (a) et lesquelles codent pour un polypeptide ayant la même séquence d'acides aminés.

12. Séquence d'ADN recombinant selon la revendication 11, dans laquelle ladite séquence d'ADN est destinée à une utilisation dans la sécurisation de l'expression d'un produit de fusion de polypeptides dans une cellule hôte procaryote ou eucaryote.

13. Premier sous domaine d'une protéine rapporteur et/ou deuxième sous domaine complémentaire d'une protéine rapporteur, dans lesquels une activité détectable de ladite protéine rapporteur est restituée quand ledit premier sous domaine et ledit deuxième sous domaine complémentaire sont placés en étroite proximité et dans lesquels ledit premier sous domaine et ledit deuxième sous domaine complémentaire ne sont pas des sous domaines de l'un des groupes de protéines constitué par des activateurs transcriptionnels, l'ubiquitine, une dihydrofolate réductase, une β-lactamase, une protéine fluorescente verte, la β-galactosidase, des intéines, une AMPc cyclase, une glycinamide ribonucléotide transformylase, une aminoglycoside phosphotransférase, une hygromycine B phosphotransférase, la luciférase ;
dans lesquels le premier sous domaine d'une protéine rapporteur ou un deuxième sous domaine complémentaire d'une protéine rapporteur est produit par un procédé de culture d'un hôte transformé avec une molécule d'ADN recombinant, choisie dans le groupe constitué par les molécules d'ADN des revendications 11 à 12, dans lesquels lesdites molécules comprennent en outre une séquence de contrôle de l'expression, ladite séquence de contrôle de l'expression étant liée, de manière opérationnelle, à ladite molécule.

14. Premier sous domaine d'une protéine rapporteur ou deuxième sous domaine complémentaire d'une protéine rapporteur selon la revendication 13, dans lequel le site de fragmentation de ladite protéine rapporteur en un premier sous domaine et un deuxième sous domaine complémentaire est identifié par un procédé selon l'une des revendications 1 à 10.

15. Protéine de fusion comprenant un premier sous domaine d'une protéine rapporteur ou un deuxième sous domaine complémentaire d'une protéine rapporteur, selon l'une des revendications 13 à 14, et un peptide ou une protéine supplémentaire relié(e) à celle-ci en une combinaison n'existant pas naturellement.

16. Lignée de cellules hôtes procaryotes ou eucaryotes transformée avec une séquence d'ADN recombinant selon l'une des revendications 11 à 12.

17. Lignée de cellules hôtes selon la revendication 16, dans laquelle la lignée de cellules hôtes permet d'obtenir une recombinaison homologue de l'ADN.

18. Lignée de cellules hôtes, selon la revendication 17, ladite lignée de cellules hôtes comprenant une lignée de cellules de levure.

19. Lignée de cellules hôtes, selon la revendication 18, qui est une lignée de cellules de levure choisie dans le groupe constitué par *Saccharomyces cerevisiae* et *Schizosaccharomyces pombe.*

20. Trousse composée de parties, comprenant un premier et un deuxième vecteurs d'expression à base d'ADN, dans laquelle :
- ledit premier vecteur d'expression contient une cassette d'expression codant pour un produit polypeptidique qui a au moins une partie substantielle de la confirmation structurale primaire d'un premier sous domaine d'une protéine rapporteur, selon l'une des revendications 13 ou 14 ; et
- ledit deuxième vecteur d'expression contient une cassette d'expression codant pour un produit polypeptidique qui a au moins une partie substantielle de la confirmation structurale primaire d'un deuxième sous domaine complémentaire d'une protéine rapporteur, selon l'une des revendications 13 ou 14 ; et
dans laquelle l'activité détectable de ladite protéine rapporteur est restituée quand ledit premier sous domaine et ledit deuxième sous domaine complémentaire sont placés en étroite proximité et dans laquelle ledit premier sous domaine et ledit deuxième sous domaine complémentaire ne sont pas des sous domaines de l'un des groupes de protéines constitué par des activateurs transcriptionnels, l'ubiquitine, une dihydrofolate réductase, une β-lactamase, une protéine fluorescente verte, la β-galactosidase, des intéines, une AMPc cyclase, une glycinamide ribonucléotide transformylase, une aminoglycoside phosphotransférase, une hygromycine B phosphotransférase, la luciférase.

21. Trousse composée de parties, selon la revendication 20, comprenant en outre une lignée appropriée de cellules hôtes procaryotes ou eucaryotes pour l'expression desdits premier et deuxième vecteurs d'expression.

22. Procédé pour détecter une interaction entre un premier peptide ou une première protéine à tester ou bien un fragment de celui-ci (celle-ci) et un deuxième peptide ou une deuxième protéine à tester ou bien un fragment de celui-ci (celle-ci), le procédé comprenant les étapes consistant à :
- fournir des séquences d'ADN recombinant, selon l'une des revendications 11 ou 12, pour être utilisées dans la sécurisation de l'expression d'un premier sous domaine d'une protéine rapporteur et d'un deuxième sous domaine complémentaire d'une protéine rapporteur ;
- fusionner un oligonucléotide ou un gène codant pour un premier peptide ou une première protéine à tester avec la séquence d'ADN codant pour ledit premier sous domaine de la protéine rapporteur, en créant de ce fait une première séquence d'ADN de fusion qui code pour une protéine de fusion comprenant ledit premier sous domaine de la protéine rapporteur et ledit premier peptide ou ladite première protéine à tester ;
- fusionner un oligonucléotide ou un gène codant pour un deuxième peptide ou une deuxième protéine à tester avec la séquence d'ADN codant pour ledit deuxième sous domaine complémentaire de la protéine rapporteur, en créant de ce fait une deuxième séquence d'ADN de fusion qui code pour une protéine de fusion comprenant ledit deuxième sous domaine complémentaire de la protéine rapporteur et ledit deuxième peptide ou ladite deuxième protéine à tester ;
- (co)-exprimer, dans une cellule hôte procaryote ou eucaryote appropriée, ladite protéine de fusion comprenant ledit premier sous domaine de la protéine rapporteur ainsi que ledit premier peptide ou ladite première protéine à tester et ladite protéine de fusion comprenant ledit deuxième sous domaine complémentaire ainsi que ledit deuxième peptide ou ladite deuxième protéine à tester ;
- effectuer un criblage et/ou une sélection en vue d'une restitution d'une activité détectable de ladite protéine rapporteur.

23. Procédé selon la revendication 22, dans lequel une banque d'oligonucléotides ou d'ADN codant pour un ensemble de premiers peptides ou de premières protéines à tester et/ou une banque d'oligonucléotides ou d'ADN codant pour un ensemble de deuxièmes peptides ou de deuxièmes protéines à tester sont fusionnées avec ledit premier sous domaine de ladite protéine rapporteur et/ou ledit deuxième sous domaine complémentaire de ladite protéine rapporteur respectivement.

24. Procédé selon l'une des revendications 22 à 23, dans lequel ledit premier peptide ou ladite première protéine à tester ou bien un fragment de celui-ci (celle-ci) et ledit deuxième peptide ou ladite deuxième protéine à tester ou bien un fragment de celui-ci (celle-ci) sont des peptides ou protéines existant naturellement dans des compartiments choisis dans le groupe constitué par des membranes cellulaires, le cytosol, la mitochondrie, le peroxysome et le lumen de la voie de sécrétion.

25. Procédé selon l'une des revendications 22 à 24, dans lequel ladite interaction entre un premier peptide ou une première protéine à tester ou bien un fragment de celui-ci (celle-ci) et un deuxième peptide ou une deuxième protéine à tester ou bien un fragment de celui-ci (celle-ci) a lieu par médiation d'un inducteur chimique de dimérisation, qui se lie de manière covalente ou non covalente auxdits peptides ou auxdites protéines à tester ou fragments de ceux-ci.

26. Procédé pour détecter l'interruption d'une interaction entre un premier peptide ou une première protéine à tester ou bien un fragment de celui-ci (celle-ci) et un deuxième peptide ou une deuxième protéine à tester ou bien un fragment de celui-ci (celle-ci), le procédé comprenant les étapes consistant à :
- fournir des séquences d'ADN recombinant, selon l'une des revendications 11 ou 12, pour une utilisation dans la sécurisation de l'expression d'un premier sous domaine d'une protéine rapporteur et un deuxième sous domaine complémentaire d'une protéine rapporteur ;
- fusionner un oligonucléotide ou un gène codant pour un premier peptide ou une première protéine à tester avec la séquence d'ADN codant pour ledit premier sous domaine de la protéine rapporteur, en créant de ce fait une première séquence d'ADN de fusion qui code pour une protéine de fusion comprenant ledit premier sous domaine de la protéine rapporteur et ledit premier peptide ou ladite première protéine à tester ;
- fusionner un oligonucléotide ou un gène codant pour un deuxième peptide ou une deuxième protéine à tester avec la séquence d'ADN codant pour ledit deuxième sous domaine complémentaire de la protéine rapporteur, en créant de ce fait une deuxième séquence d'ADN de fusion qui code pour une protéine de fusion comprenant ledit deuxième sous domaine complémentaire de la protéine rapporteur et ledit deuxième peptide ou ladite deuxième protéine à tester ;
- (co)-exprimer, dans une cellule hôte procaryote ou eucaryote appropriée, ladite protéine de fusion comprenant ledit premier sous domaine de la protéine rapporteur ainsi que ledit premier peptide ou ladite première protéine à tester et ladite protéine de fusion comprenant ledit deuxième sous domaine complémentaire de la protéine rapporteur ainsi que ledit deuxième peptide ou ladite deuxième protéine à tester ;
- effectuer un criblage et/ou une sélection en vue d'une interruption de l'interaction dudit premier sous domaine et dudit deuxième sous domaine étant sous l'influence d'un ou plusieurs agent(s) de test.

27. Utilisation d'une permutation circulaire aléatoire d'un gène et/ou du polypeptide exprimé dérivé de celui-ci pour l'identification de sites de fragmentation dans une protéine rapporteur destinée à être utilisée dans un système à deux hybrides.

28. Utilisation d'une lignée de cellules hôtes capable d'une recombinaison homologue d'ADN dans un procédé selon l'une des revendications 1 à 10.


### SEQUENCE LISTING

<110> Ecole Polytechnique Fédérale de Lausanne (EPFL)
<120> Method for identification of suitable fragmentation sites in a reporter protein
<130> PEPF001WO
<150> US 34,404 JM-213
   <151> 2003-10-09
<160> 66
<170> PatentIn version 3.1
<210> 1
   <211> 672
   <212> DNA
   <213> Saccharomyces cerevisiae
<220>
   <221> modified_base
   <222> (186)..(186)
   <223> silent point mutation introduced to generate HindIII restriction site
<300>
   <308> NCBI / NC_001136
   <309> 2004-08-30
<400> 1
   atgtctgtta ttaatttcac aggtagttct ggtccattgg tgaaagtttg cggcttgcag      60
   agcacagagg ccgcagaatg tgctctagat tccgatgctg acttgctggg tattatatgt     120
   gtgcccaata gaaagagaac aattgacccg gttattgcaa ggaaaatttc aagtcttgta     180
   aaagcatata aaaatagttc aggcactccg aaatacttgg ttggcgtgtt tcgtaatcaa     240
   cctaaggagg atgttttggc tctggtcaat gattacggca ttgatatcgt ccaactgcac     300
   ggagatgagt cgtggcaaga ataccaagag ttcctcggtt tgccagttat taaaagactc     360
   gtatttccaa aagactgcaa catactactc agtgcagctt cacagaaacc tcattcgttt     420
   attcccttgt ttgattcaga agcaggtggg acaggtgaac ttttggattg gaactcgatt     480
   tctgactggg ttggaaggca agagagcccc gagagcttac attttatgtt agctggtgga     540
   ctgacgccag aaaatgttgg tgatgcgctt agattaaatg gcgttattgg tgttgatgta     600
   agcggaggtg tggagacaaa tggtgtaaaa gactctaaca aaatagcaaa tttcgtcaaa     660
   aatgctaaga aa                                                         672
<210> 2
   <211> 224
   <212> PRT
   <213> Saccharomyces cerevisiae
<300>
   <308> NCBI / NC_O01136
   <309> 2004-08-30
<400> 2
   Met Ser Val Ile Asn Phe Thr Gly Ser Ser Gly Pro Leu Val Lys Val
   1               5                   10                  15
   Cys Gly Leu Gln Ser Thr Glu Ala Ala Glu Cys Ala Leu Asp Ser Asp
               20                  25                  30
   Ala Asp Leu Leu Gly Ile Ile Cys Val Pro Asn Arg Lys Arg Thr Ile
           35                  40                  45
   Asp Pro Val Ile Ala Arg Lys Ile Ser Ser Leu Val Lys Ala Tyr Lys
       50                  55                  60
   Asn Ser Ser Gly Thr Pro Lys Tyr Leu Val Gly Val Phe Arg Asn Gln
   65                  70                  75                  80
   Pro Lys Glu Asp Val Leu Ala Leu Val Asn Asp Tyr Gly Ile Asp Ile
                   85                  90                  95
   Val Gln Leu His Gly Asp Glu Ser Trp Gln Glu Tyr Gln Glu Phe Leu
               100                 105                 110
   Gly Leu Pro Val Ile Lys Arg Leu Val Phe Pro Lys Asp Cys Asn Ile
           115                 120                 125
   Leu Leu Ser Ala Ala Ser Gln Lys Pro His Ser Phe Ile Pro Leu Phe
       130                 135                 140
   Asp Ser Glu Ala Gly Gly Thr Gly Glu Leu Leu Asp Trp Asn Ser Ile
   145                 150                 155                 160
   Ser Asp Trp Val Gly Arg Gln Glu Ser Pro Glu Ser Leu His Phe Met
                   165                 170                 175
   Leu Ala Gly Gly Leu Thr Pro Glu Asn Val Gly Asp Ala Leu Arg Leu
               180                 185                 190
   Asn Gly Val Ile Gly Val Asp Val Ser Gly Gly Val Glu Thr Asn Gly
           195                 200                 205
   Val Lys Asp Ser Asn Lys Ile Ala Asn Phe Val Lys Asn Ala Lys Lys
       210                 215                 220
<210> 3
   <211> 132
   <212> DNA
   <213> Saccharomyces cerevisiae
<220>
   <221> modified_base
   <222> (22)..(22)
   <223> point mutation
<400> 3
   atgtctgtta ttaatttcac atgtagttct ggtccattgg tgaaagtttg cggcttgcag     60
   agcacagagg ccgcagaatg tgctctagat tccgatgctg acttgctggg tattatatgt    120
   gtgcccaata ga                                                        132
<210> 4
   <211> 44
   <212> PRT
   <213> Saccharomyces cerevisiae
<400> 4
   Met Ser Val Ile Asn Phe Thr Cys Ser Ser Gly Pro Leu Val Lys Val
   1               5                   10                  15
   Cys Gly Leu Gln Ser Thr Glu Ala Ala Glu Cys Ala Leu Asp Ser Asp
               20                  25                  30
   Ala Asp Leu Leu Gly Ile Ile Cys Val Pro Asn Arg
           35                  40
<210> 5
   <211> 540
   <212> DNA
   <213> Saccharomyces cerevisiae
<400> 5
   aagagaacaa ttgacccggt tattgcaagg aaaatttcaa gtcttgtaaa agcatataaa     60
   aatagttcag gcactccgaa atacttggtt ggcgtgtttc gtaatcaacc taaggaggat    120
   gttttggctc tggtcaatga ttacggcatt gatatcgtcc aactgcacgg agatgagtcg    180
   tggcaagaat accaagagtt cctcggtttg ccagttatta aaagactcgt atttccaaaa    240
   gactgcaaca tactactcag tgcagcttca cagaaacctc attcgtttat tcccttgttt    300
   gattcagaag caggtgggac aggtgaactt ttggattgga actcgatttc tgactgggtt    360
   ggaaggcaag agagccccga gagcttacat tttatgttag ctggtggact gacgccagaa    420
   aatgttggtg atgcgcttag attaaatggc gttattggtg ttgatgtaag cggaggtgtg    480
   gagacaaatg gtgtaaaaga ctctaacaaa atagcaaatt tcgtcaaaaa tgctaagaaa    540
<210> 6
   <211> 180
   <212> PRT
   <213> Saccharomyces cerevisiae
<400> 6
   Lys Arg Thr Ile Asp Pro Val Ile Ala Arg Lys Ile Ser Ser Leu Val
   1 5 10 15
   Lys Ala Tyr Lys Asn Ser Ser Gly Thr Pro Lys Tyr Leu Val Gly Val
   20 25 30
   Phe Arg Asn Gln Pro Lys Glu Asp Val Leu Ala Leu Val Asn Asp Tyr
   35 40 45
   Gly Ile Asp Ile Val Gln Leu His Gly Asp Glu Ser Trp Gln Glu Tyr
   50 55 60
   Gln Glu Phe Leu Gly Leu Pro Val Ile Lys Arg Leu Val Phe Pro Lys
   65 70 75 80
   Asp Cys Asn Ile Leu Leu Ser Ala Ala Ser Gln Lys Pro His Ser Phe
   85 90 95
   Ile Pro Leu Phe Asp Ser Glu Ala Gly Gly Thr Gly Glu Leu Leu Asp
   100 105 110
   Trp Asn Ser Ile Ser Asp Trp Val Gly Arg Gln Glu Ser Pro Glu Ser
   115 120 125
   Leu His Phe Met Leu Ala Gly Gly Leu Thr Pro Glu Asn Val Gly Asp
   130 135 140
   Ala Leu Arg Leu Asn Gly Val Ile Gly Val Asp Val Ser Gly Gly Val
   145 150 155 160
   Glu Thr Asn Gly Val Lys Asp Ser Asn Lys Ile Ala Asn Phe Val Lys
   165 170 175
   Asn Ala Lys Lys
   180
<210> 7
   <211> 159
   <212> DNA
   <213> Saccharomyces cerevisiae
<400> 7
   atgtctgtta ttaatttcac aggtagttct ggtccattgg tgaaagtttg cggcttgcag 60
   agcacagagg ccgcagaatg tgctctagat tccgatgctg acttgctggg tattatatgt 120
   gtgcccaata gaaagagaac aattgacccg gttattgca 159
<210> 8
   <211> 53
   <212> PRT
   <213> Saccharomyces cerevisiae
<400> 8
   Met Ser Val Ile Asn Phe Thr Gly Ser Ser Gly Pro Leu Val Lys Val
   1               5                   10                  15
   Cys Gly Leu Gln Ser Thr Glu Ala Ala Glu Cys Ala Leu Asp Ser Asp
               20                  25                  30
   Ala Asp Leu Leu Gly Ile Ile Cys Val Pro Asn Arg Lys Arg Thr Ile
           35                  40                  45
   Asp Pro Val Ile Ala
       50
<210> 9
   <211> 516
   <212> DNA
   <213> Saccharomyces cerevisiae
<400> 9
   gcaaggaaaa tttcaagtct tgtaaaagct tataaaaata gttcaggcac tccgaaatac    60
   ttggttggcg tgtttcgtaa tcaacctaag gaggatgttt tggctctggt caatgattac   120
   ggcattgata tcgtccaact gcacggagat gagtcgtggc aagaatacca agagttcctc   180
   ggtttgccag ttattaaaag actcgtattt ccaaaagact gcaacatact actcagtgca   240
   gcttcacaga aacctcattc gtttattccc ttgtttgatt cagaagcagg tgggacaggt   300
   gaacttttgg attggaactc gatttctgac tgggttggaa ggcaagagag ccccgagagc   360
   ttacatttta tgttagctgg tggactgacg ccagaaaatg ttggtgatgc gcttagatta   420
   aatggcgtta ttggtgttga tgtaagcgga ggtgtggaga caaatggtgt aaaagactct   480
   aacaaaatag caaatttcgt caaaaatgct aagaaa                             516
<210> 10
   <211> 172
   <212> PRT
   <213> Saccharomyces cerevisiae
<400> 10
   Ala Arg Lys Ile Ser Ser Leu Val Lys Ala Tyr Lys Asn Ser Ser Gly
   1               5                   10                  15
   Thr Pro Lys Tyr Leu Val Gly Val Phe Arg Asn Gln Pro Lys Glu Asp
               20                  25                  30
   Val Leu Ala Leu Val Asn Asp Tyr Gly Ile Asp Ile Val Gln Leu His
           35                  40                  45
   Gly Asp Glu Ser Trp Gln Glu Tyr Gln Glu Phe Leu Gly Leu Pro Val
       50                  55                  60
   Ile Lys Arg Leu Val Phe Pro Lys Asp Cys Asn Ile Leu Leu Ser Ala
   65                  70                  75                  80
   Ala Ser Gln Lys Pro His Ser Phe Ile Pro Leu Phe Asp Ser Glu Ala
                   85                  90                  95
   Gly Gly Thr Gly Glu Leu Leu Asp Trp Asn Ser Ile Ser Asp Trp Val
               100                 105                 110
   Gly Arg Gln Glu Ser Pro Glu Ser Leu His Phe Met Leu Ala Gly Gly
           115                 120                 125
   Leu Thr Pro Glu Asn Val Gly Asp Ala Leu Arg Leu Asn Gly Val Ile
       130                 135                 140
   Gly Val Asp Val Ser Gly Gly Val Glu Thr Asn Gly Val Lys Asp Ser
   145                 150                 155                 160
   Asn Lys Ile Ala Asn Phe Val Lys Asn Ala Lys Lys
                   165                 170
<210> 11
   <211> 561
   <212> DNA
   <213> Saccharomyces cerevisiae
<400> 11
   atgtctgtta ttaatttcac aggtagttct ggtccattgg tgaaagtttg cggcttgcag 60
   agcacagagg ccgcagaatg tgctctagat tccgatgctg acttgctggg tattatatgt 120
   gtgcccaata gaaagagaac aattgacccg gttattgcaa ggaaaatttc aagtcttgta 180
   aaagcttata aaaatagttc aggcactccg aaatacttgg ttggcgtgtt tcgtaatcaa 240
   cctaaggagg atgttttggc tctggtcaat gattacggca ttgatatcgt ccaactgcac 300
   ggagatgagt cgtggcaaga ataccaagag ttcctcggtt tgccagttat taaaagactc 360
   gtatttccaa aagactgcaa catactactc agtgcagctt cacagaaacc tcattcgttt 420
   attcccttgt ttgattcaga agcaggtggg acaggtgaac ttttggattg gaactcgatt 480
   tctgactggg ttggaaggca agagagcccc gagagcttac attttatgtt agctggtgga 540
   ctgacgccag aaaatgttgg t 561
<210> 12
   <211> 187
   <212> PRT
   <213> Saccharomyces cerevisiae
<400> 12
   Met Ser Val Ile Asn Phe Thr Gly Ser Ser Gly Pro Leu Val Lys Val
   1 5 10 15
   Cys Gly Leu Gln Ser Thr Glu Ala Ala Glu Cys Ala Leu Asp Ser Asp
   20 25 30
   Ala Asp Leu Leu Gly Ile Ile Cys Val Pro Asn Arg Lys Arg Thr Ile
   35 40 45
   Asp Pro Val Ile Ala Arg Lys Ile Ser Ser Leu Val Lys Ala Tyr Lys
   50 55 60
   Asn Ser Ser Gly Thr Pro Lys Tyr Leu Val Gly Val Phe Arg Asn Gln
   65 70 75 80
   Pro Lys Glu Asp Val Leu Ala Leu Val Asn Asp Tyr Gly Ile Asp Ile
   85 90 95
   Val Gln Leu His Gly Asp Glu Ser Trp Gln Glu Tyr Gln Glu Phe Leu
   100 105 110
   Gly Leu Pro Val Ile Lys Arg Leu Val Phe Pro Lys Asp Cys Asn Ile
   115 120 125
   Leu Leu Ser Ala Ala Ser Gln Lys Pro His Ser Phe Ile Pro Leu Phe
   130 135 140
   Asp Ser Glu Ala Gly Gly Thr Gly Glu Leu Leu Asp Trp Asn Ser Ile
   145 150 155 160
   Ser Asp Trp Val Gly Arg Gln Glu Ser Pro Glu Ser Leu His Phe Met
   165 170 175
   Leu Ala Gly Gly Leu Thr Pro Glu Asn Val Gly
   180 185
<210> 13
   <211> 111
   <212> DNA
   <213> Saccharomyces cerevisiae
<400> 13
   gatgcgctta gattaaatgg cgttattggt gttgatgtaa gcggaggtgt ggagacaaat 60
   ggtgtaaaag actctaacaa aatagcaaat ttcgtcaaaa atgctaagaa a 111
<210> 14
   <211> 37
   <212> PRT
   <213> Saccharomyces cerevisiae
<400> 14
   Asp Ala Leu Arg Leu Asn Gly Val Ile Gly Val Asp Val Ser Gly Gly
   1               5                   10                  15
   Val Glu Thr Asn Gly Val Lys Asp Ser Asn Lys Ile Ala Asn Phe Val
               20                  25                  30
   Lys Asn Ala Lys Lys
           35
<210> 15
   <211> 612
   <212> DNA
   <213> Saccharomyces cerevisiae
<220>
   <221> mutation
   <222> (22) .. (22)
   <223> point mutation
<220>
   <221> deletion
   <222> (612)..(612)
   <223> missing sequence after base 612 of wild-type: GAGACAAATGGTGTAAAAG ACTCT
<400> 15
   atgtctgtta ttaatttcac atgtagttct ggtccattgg tgaaagtttg cggcttgcag      60
   agcacagagg ccgcagaatg tgctctagat tccgatgctg acttgctggg tattatatgt     120
   gtgcccaata gaaagagaac aattgacccg gttattgcaa ggaaaatttc aagtcttgta     180
   aaagcttata aaaatagttc aggcactccg aaatacttgg ttggcgtgtt tcgtaatcaa     240
   cctaaggagg atgttttggc tctggtcaat gattacggca ttgatatcgt ccaactgcac     300
   ggagatgagt cgtggcaaga ataccaagag ttcctcggtt tgccagttat taaaagactc     360
   gtatttccaa aagactgcaa catactactc agtgcagctt cacagaaacc tcattcgttt     420
   attcccttgt ttgattcaga agcaggtggg acaggtgaac ttttggattg gaactcgatt     480
   tctgactggg ttggaaggca agagagcccc gagagcttac attttatgtt agctggtgga     540
   ctgacgccag aaaatgttgg tgatgcgctt agattaaatg gcgttattgg tgttgatgta 600
   agcggaggtg tg 612
<210> 16
   <211> 204
   <212> PRT
   <213> Saccharomyces cerevisiae
<400> 16
   Met Ser Val Ile Asn Phe Thr Cys Ser Ser Gly Pro Leu Val Lys Val
   1               5                   10                  15
   Cys Gly Leu Gln Ser Thr Glu Ala Ala Glu Cys Ala Leu Asp Ser Asp
               20                  25                  30
   Ala Asp Leu Leu Gly Ile Ile Cys Val Pro Asn Arg Lys Arg Thr Ile
           35                  40                  45
   Asp Pro Val Ile Ala Arg Lys Ile Ser Ser Leu Val Lys Ala Tyr Lys
       50                  55                  60
   Asn Ser Ser Gly Thr Pro Lys Tyr Leu Val Gly Val Phe Arg Asn Gln
   65                  70                  75                  80
   Pro Lys Glu Asp Val Leu Ala Leu Val Asn Asp Tyr Gly Ile Asp Ile
                   85                  90                  95
   Val Gln Leu His Gly Asp Glu Ser Trp Gln Glu Tyr Gln Glu Phe Leu
               100                 105                 110
   Gly Leu Pro Val Ile Lys Arg Leu Val Phe Pro Lys Asp Cys Asn Ile
           115                 120                 125
   Leu Leu Ser Ala Ala Ser Gln Lys Pro His Ser Phe Ile Pro Leu Phe
       130                 135                 140
   Asp Ser Glu Ala Gly Gly Thr Gly Glu Leu Leu Asp Trp Asn Ser Ile
   145                 150                 155                 160
   Ser Asp Trp Val Gly Arg Gln Glu Ser Pro Glu Ser Leu His Phe Met
                   165                 170                 175
   Leu Ala Gly Gly Leu Thr Pro Glu Asn Val Gly Asp Ala Leu Arg Leu
               180                 185                 190
   Asn Gly Val Ile Gly Val Asp Val Ser Gly Gly Val
           195                 200
<210> 17
   <211> 36
   <212> DNA
   <213> Saccharomyces cerevisiae
<220>
   <221> mutation
   <222> (1)..(1)
   <223> missing sequence before base 1 of SEQ17, corresponding to base 63 7 of wild-type: GAGACAAATGGTGTAAAAGACTCT
<400> 17
   aacaaaatag caaatttcgt caaaaatgct aagaaa 36
<210> 18
   <211> 12
   <212> PRT
   <213> Saccharomyces cerevisiae
<400> 18
   Asn Lys Ile Ala Asn Phe Val Lys Asn Ala Lys Lys
   1 5 10
<210> 19
   <211> 43
   <212> PRT
   <213> Artificial Sequence
<220>
   <223> first of a pair of peptides (together with peptide C2), that asso ciate into an anti-parallel coiled coil (Biochemistry 37 (1998), 12603-12610)
<400> 19
   Met Asp Tyr Lys Asp Glu Ser Gly Gln Ala Leu Glu Lys Glu Leu Ala
   1 5 10 15
   Gln Asn Glu Trp Glu Leu Gln Ala Leu Glu Lys Glu Leu Ala Gln Leu
   20 25 30
   Glu Lys Glu Leu Gln Ala Gly Ser Gly Ser Gly
   35 40
<210> 20
   <211> 50
   <212> PRT
   <213> Artificial Sequence
<220>
   <223> second of a pair of peptides (together with peptide C1), that ass ociate into an anti-parallel coiled coil (Biochemistry 37 (1998), 12603-12610)
<400> 20
   Gly Gly Ser Gly Ser Gly Gln Ala Leu Lys Lys Lys Leu Ala Gln Leu
   1               5                   10                  15
   Lys Trp Lys Leu Gln Ala Leu Lys Lys Lys Asn Ala Gln Leu Lys Lys
               20                  25                  30
   Lys Leu Gln Ala Gly Ser Tyr Pro Tyr Asp Val Pro Asp Tyr Ala Ala
           35                  40                  45
   Phe Leu
       50
<210> 21
   <211> 39
   <212> DNA
   <213> Artificial Sequence
<220>
   <223> Primer for Ctrp fusions
<400> 21
   cgatacgaat tcatggacaa ggattgtgaa atgaaacgc 39
<210> 22
   <211> 45
   <212> DNA
   <213> Artificial Sequence
<220>
   <223> Primer for pRS316-C1/2CUP1
<400> 22
   aaaggaattg gcccaaaatg agtgggagtt acaagcactt gagaa 45
<210> 23
   <211> 45
   <212> DNA
   <213> Artificial Sequence
<220>
   <223> Primer for pRS316-C1/2CUP1
<400> 23
   ctcaatgttc gtgaactctt cctcgagcga gttgaactct tcctc 45
<210> 24
   <211> 45
   <212> DNA
   <213> Artificial Sequence
<220>
   <223> Primer for pRS316-C1/2CUP1
<400> 24
   ctccttctca agttgagcga gctccttctc aagtgcttgt aactc 45
<210> 25
   <211> 42
   <212> DNA
   <213> Artificial Sequence
<220>
   <223> Primer for pRS316-C1/2CUP1
<400> 25
   ggcacttaag aagaagttgg cgcagcttaa gtggaaactg ca 42
<210> 26
   <211> 46
   <212> DNA
   <213> Artificial Sequence
<220>
   <223> Primer for pRS316-C1/2CUP1
<400> 26
   agctgggcat tcttcttctt aagagcttgc agtttccact taagct 46
<210> 27
   <211> 48
   <212> DNA
   <213> Artificial Sequence
<220>
   <223> Primer for pRS316-C1/2CUP1
<400> 27
   aagaagaaga atgcccagct taagaagaag ctccaggctg gaagttac 48
<210> 28
   <211> 45
   <212> DNA
   <213> Artificial Sequence
<220>
   <223> Primer for pRS316-C1/2CUP1
<400> 28
   atacgatgtt ccagattacg ctgcattttt ataagtcgac tggtc 45
<210> 29
   <211> 22
   <212> DNA
   <213> Artificial Sequence
<220>
   <223> Primer for pRS316-C1/2CUP1
<400> 29
   gaccagtcga cttataaaaa tg 22
<210> 30
   <211> 25
   <212> DNA
   <213> Artificial Sequence
<220>
   <223> Primer for circular permutation of TRP1
<400> 30
   gtaaaagctt ataaaaatag ttcag 25
<210> 31
   <211> 36
   <212> DNA
   <213> Artificial Sequence
<220>
   <223> Primer for circular permutation of TRP1
<400> 31
   gaaatagcct aggatgtctg ttattaattt cacagg 36
<210> 32
   <211> 35
   <212> DNA
   <213> Artificial Sequence
<220>
   <223> Primer for circular permutation of TRP1
<400> 32
   cagacatcct aggctatttc ttagcatttt tgacg 35
<210> 33
   <211> 25
   <212> DNA
   <213> Artificial Sequence
<220>
   <223> Primer for circular permutation of-TRP1
<400> 33
   tttataagct tttacaagac ttgaa 25
<210> 34
   <211> 22
   <212> DNA
   <213> Artificial Sequence
<220>
   <223> Primer for pRS316-C1/2CUP1
<400> 34
   gtaacgaatt catggactac aa 22
<210> 35
   <211> 66
   <212> DNA
   <213> Artificial Sequence
<220>
   <223> Primer for pRS316-C1/2CUP1
<400> 35
   cattttgggc caattccttt tccagtgctt gaccactttc gtctttgtag tccatgaatt 60
   cgttac 66
<210> 36
   <211> 64
   <212> DNA
   <213> Artificial Sequence
<220>
   <223> Primer for pRS316-C1/2CUP1
<400> 36
   caacttcttc ttaagtgcct gacctgatcc agatccaccg ttaacacctg aacctgatcc 60
   ggcc 64
<210> 37
   <211> 43
   <212> DNA
   <213> Artificial Sequence
<220>
   <223> Primer for pRS316-C1/2CUP1
<400> 37
   gtaatctgga acatcgtatg ggtaacttcc agcctggagc ttc 43
<210> 38
   <211> 66
   <212> DNA
   <213> Artificial Sequence
<220>
   <223> Primer for homologous recombination
<400> 38
   gactctaaca aaatagcaaa tttcgtcaaa aatgctaaga aatagagggc cgcatcatgt 60
   aattag 66
<210> 39
   <211> 65
   <212> DNA
   <213> Artificial Sequence
<220>
   <223> Primer for homologous recombination
<400> 39
   aactttcacc aatggaccag aactacctgt gaaattaata acagacattt tgagatccgg 60
   gtttt 65
<210> 40
   <211> 40
   <212> DNA
   <213> Artificial Sequence
<220>
   <223> Primer for Ctrp fusions
<400> 40
   gcctgatcca gatccgcctt ctggtgattc atcatcttca 40
<210> 41
   <211> 40
   <212> DNA
   <213> Artificial Sequence
<220>
   <223> Primer for Ctrp fusions
<400> 41
   ggcggatctg gatcaggcaa gagaacaatt gacccggtta 40
<210> 42
   <211> 40
   <212> DNA
   <213> Artificial Sequence
<220>
   <223> Primer for Ctrp fusions
<400> 42
   gcctgatcca gatccgccta taaaaggtat tccgacacca 40
<210> 43
   <211> 39
   <212> DNA
   <213> Artificial Sequence
<220>
   <223> Primer for Ntrp fusions
<400> 43
   gcctgatcca gatccgcctg caataaccgg gtcaattgt 39
<210> 44
   <211> 40
   <212> DNA
   <213> Artificial Sequence
<220>
   <223> Primer for Ctrp fusions
<400> 44
   ggcggatctg gatcaggcgc aaggaaaatt tcaagtcttg 40
<210> 45
   <211> 42
   <212> DNA
   <213> Artificial Sequence
<220>
   <223> Primer for Ntrp fusions
<400> 45
   gcctgatcca gatccgccac caacattttc tggcgtcagt cc 42
<210> 46
   <211> 39
   <212> DNA
   <213> Artificial Sequence
<220>
   <223> Primer for Ctrp fusions
<400> 46
   ggcggatctg gatcaggcga tgcgcttaga ttaaatggc 39
<210> 47
   <211> 39
   <212> DNA
   <213> Artificial Sequence
<220>
   <223> Primer for Ntrp fusions
<400> 47
   gcctgatcca gatccgccca cacctccgct tacatcaac 39
<210> 48
   <211> 28
   <212> DNA
   <213> Artificial Sequence
<220>
   <223> Primer for Ctrp fusions
<400> 48
   ggcggatctg gatcaggcaa caaaatag 28
<210> 49
   <211> 25
   <212> DNA
   <213> Artificial Sequence
<220>
   <223> Primer for pRS315CUP1/ pRS316CUP1
<400> 49
   agcaggatcc cattaccgac atttg 25
<210> 50
   <211> 41
   <212> DNA
   <213> Artificial Sequence
<220>
   <223> Primer for pRS315CUP1/ pRS316CUP1
<400> 50
   cctaggttga gatctcttga attcgttaca gtttgttttt c 41
<210> 51
   <211> 43
   <212> DNA
   <213> Artificial Sequence
<220>
   <223> Primer for pRS315CUP1/ pRS316CUP1
<400> 51
   aattcaagag atctcaacct aggatgacgg tgggaggtct ata 43
<210> 52
   <211> 45
   <212> DNA
   <213> Artificial Sequence
<220>
   <223> Primer for pRS315CUP1/ pRS316CUP1
<400> 52
   cgattgtcga cggtcgtacg ctaacgcttc tcgttggggt ctttg 45
<210> 53
   <211> 36
   <212> DNA
   <213> Artificial Sequence
<220>
   <223> Primer for Ctrp fusions
<400> 53
   acttgtcgac tcagttttgt tcggcttttt cattga 36
<210> 54
   <211> 33
   <212> DNA
   <213> Artificial Sequence
<220>
   <223> Primer for Ctrp fusions
<400> 54
   agatagatct atgcctacaa attacgagta tga 33
<210> 55
   <211> 60
   <212> DNA
   <213> Artificial Sequence
<220>
   <223> Primer for Ntrp fusions
<400> 55
   agatagatct atgcatcatc atcatcatca cagcagctct gttattaatt tcacaggtag 60
<210> 56
   <211> 59
   <212> DNA
   <213> Artificial Sequence
<220>
   <223> Primer for Ntrp fusions
<400> 56
   acctgtcgac ctagtgatga tgatgatgat gacttttctt agcatttttg acgaaattt 59
<210> 57
   <211> 33
   <212> DNA
   <213> Artificial Sequence
<220>
   <223> Primer for Ctrp fusions
<400> 57
   agtaagatct atgcaccaag attttcaaga aga 33
<210> 58
   <211> 68
   <212> DNA
   <213> Artificial Sequence
<220>
   <223> Primer for Ctrp fusions
<400> 58
   gtaagtcgac ttaagcgtaa tctggaacat cgtatgggta ggatttctta gcatttttga 60
   cgaaattt 68
<210> 59
   <211> 66
   <212> DNA
   <213> Artificial Sequence
<220>
   <223> Primer for Ntrp fusions
<400> 59
   caagagatct atgtacccat acgatgttcc agattacgct tctatgtctg ttattaattt 60
   cacagg 66
<210> 60
   <211> 38
   <212> DNA
   <213> Artificial Sequence
<220>
   <223> Primer for Ntrp fusions
<400> 60
   ggcggatctg gatcaggcat gatcagtctg attgcggc 38
<210> 61
   <211> 29
   <212> DNA
   <213> Artificial Sequence
<220>
   <223> Primer for Ntrp fusions
<400> 61
   cgaggtcgac ttaccgccgc tccagaatc 29
<210> 62
   <211> 30
   <212> DNA
   <213> Artificial Sequence
<220>
   <223> Primer for Ctrp fusions
<400> 62
   gcctgatcca gatccgccgt ttcggccagc 30
<210> 63
   <211> 34
   <212> DNA
   <213> artificial sequence
<220>
   <223> Primer for homologous recombination
<400> 63
   gtggcctatg ctaatgcagg gccgcaaatt aaag 34
<210> 64
   <211> 32
   <212> DNA
   <213> Artificial sequence
<220>
   <223> Primer used for homologous recombination
<400> 64
   ccctgcatta gcataggcca ctagtggatc tg 32
<210> 65
   <211> 41
   <212> DNA
   <213> Artificial Sequence
<220>
   <223> Primer used for N-trp cloning, Sec62 cloning
<400> 65
   gcctgatcca gatccgcctc tattgggcac acatataata c 41
<210> 66
   <211> 38
   <212> DNA
   <213> Artificial Sequence
<220>
   <223> Primer used for N-trp cloning, Sec62 cloning
<400> 66
   ggcggatctg gatcaggcat ggtagccgag caaacaca 38